# EUROPEAN PATENT SPECIFICATION

(11) **EP 0 464 558 B1**
(45) Date of publication and mention of the grant of the patent: **28.12.1994**
(21) Application number: 91110376.0
(22) Date of filing: 24.06.1991
(51) Int. Cl.: C07D 403/12, C07D 403/14, A61K 31/40, A61K 31/505

(54) **Antimigraine alkoxypyrimidine derivatives**
Antimigräne Alkoxypyrimidinderivate
Dérivés antimigraines d'alkoxypyrimidine

(30) Priority: 29.06.1990 US 546122; 04.04.1991 US 680208
(43) Date of publication of application: 08.01.1992
(73) Proprietor: Bristol-Myers Squibb Company, New York, N.Y. 10154 (US)
(72) Inventor: Smith, David W., Clinton, Connecticut 06413 (US); Yocca, Frank D., Madison, Connecticut 06443 (US); Yevich, Joseph P., Southington, Connecticut 06489 (US); Mattson, Ronald J., Meriden, Connecticut 06450 (US)
(74) Representative: Kinzebach, Werner, Dr.

(56) References cited:
- EP-A- 0 242 939
- EP-A- 0 345 808
- DE-B- 2 110 534
- US-A- 3 562 278
- CHEMICAL ABSTRACTS, vol. 93, no. 23, 08 December 1980 Columbus, Ohio, USA Trubitsyna,T.K. et al.: "Pharmacological properties of some indolylalkyl derivatives of & Farmakol.Toksikol.[Moscow] 1980, vol.43,no.5,pages 530-6.[Russ]. page 18; column 1; ref. no. 215274Y

## Description

This invention generally pertains to heterocyclic carbon compounds having drug and bio-affecting properties and to their preparation and use. In particular the invention is concerned with 1,4-disubstituted piperazine derivatives wherein one substituent is indol-3-yl-alkyl and the other is an alkoxy-substituted pyrimidin-4-yl moiety. These compounds possess a unique serotonergic profile that renders them useful in treatment of vascular headaches of the migraine type.

Archer disclosed a large series of CNS-depressant indolylalkylpiperazines in U.S. 3,188,313. Among a large number of possible substituents on the 4-nitrogen atom of the piperazine ring was pyrimidine (unsubstituted). In U.S. 3,562,278, Archer disclosed and claimed a series of 1-indolylethyl-4-substituted- piperazines. Among the possible 4-substituents listed is 2-pyrimidinyl, again unsubstituted. The pharmacologic action disclosed for these art compounds is general CNS and psychomotor depression which bear no relationship to an antimigraine effect. Thus the compounds of Archer do not suggest the alkoxypyrimidine antimigraine compounds of this invention.

Dowie, et al. disclosed a series of 3-alkylaminoindole derivatives as being potentially useful for the treatment of migraine in a published patent application, GB 2,124,210. One member of this series of compounds was specifically claimed in a later patent application of Oxford, GB 2,162,522, published February 5, 1986. This particular compound is known in the literature as sumatriptan.(1 )

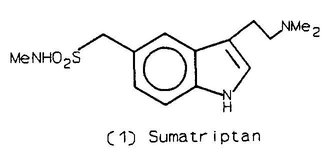

A series of novel indoline derivatives were disclosed February 7, 1990 by Manoury, et al., in European patent application EPA 354,094. These compounds are described as being useful for treatment of various CNS disorders including depression, anxiety and migraine. Included among these art compounds are those of formula (2). 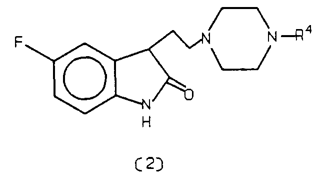 wherein R⁴ is aryl, pyridine or quinoline moieties.

None of these art compounds make obvious the instant novel alkoxypyrimidine derivatives for treatment of migraine.

Migraine is a member of a broader class of headache which also comprises cluster headaches and other headaches believed to have a vascular implication in their etiology. These headaches are classified as vascular headaches. For a current summary of headache and its treatment see: Chapter 13: "Drugs Used to Treat Migraine and Other Headaches" in Drug Evaluations, 6th Edn., 1986, pages 239-253 American Medical Association, W.B. Saunders Co., Philadephia, PA.

Frequent irregularly-occurring episodes of headache afflict a large number of people but are usually acute in nature and of short duration. Relief of this type of headache is typically provided by mild analgesics such as aspirin or acetaminophen. Such headaches are quite common and, while painful and perhaps annoying, are seldom incapacitating and debilitating. Chronic recurrent headaches of the vascular category, however, usually lead to patient consultation with a physician due to pain severity which is often incapacitating.

Although there is no universally accepted classification system for headache, vascular headache, for the purposes of the present invention, refers mainly to migraine and cluster headaches. Migraine includes the common or classical type as well as migraine variants which would be familiar to one skilled in the art. Other subtypes such as toxic vascular and hypertensive headaches, as well as some muscle-contraction and combined or mixed vascular-muscle headaches may also fall into a vascular-related headache category and be treatable by the present invention. It is appreciated by one skilled in the art that no single therapy is effective in all patients diagnosed with the same subtype of headache, thereby raising further uncertainties about headache classification.

Drugs most commonly used in treatment of headache fall into the following groups:
Ergot Alkaloids,
Beta-blocking Agents,
Calcium Channel Blocking Agents,
Antidepressants, and
Mixtures of these.

Management of recurring vascular headache is complicated by the lack of a single therapy which is effective in all patients with the same headache type and by the need to select either an abortive or prophylactic method of treatment for these headaches. Further complication involves the current use of drugs that cause dependence with extended use, such as ergotamine. Another important consideration for the present invention is that the more effective antimigraine agents in current use, e.g. the ergots, methysergide, produce severe use-limiting side-effects with long term usage.

EP-A-345808 discloses 1,4-disubstituted piperazine derivatives of the formula: 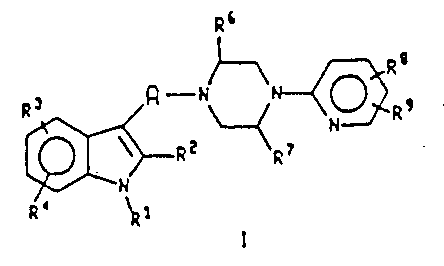 wherein
R¹ and R² are independently selected from lower, meaning C, -C₄ alkyl or hydrogen;
R³, R⁴, R⁸ and R⁹ are independently selected from hydrogen, lower alkyl, lower alkoxy, carbamide, halogen, trifluoromethyl and thio-lower alkyl, with the proviso that R⁸ and R⁹ cannot both be hydrogen at the same time;
A is a C₅-₇ cycloalkanyl or cycloalkenyl ring, or A is 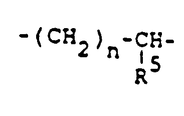 wherein n is an integer from 1 to 3 and R⁵ is the same as R¹;
and R⁶ and R⁷ are independently selected from hydrogen, methyl or R⁶ and R⁷ can be taken together as a methylene bridge.

EP-A-242939 discloses compounds of the formula: 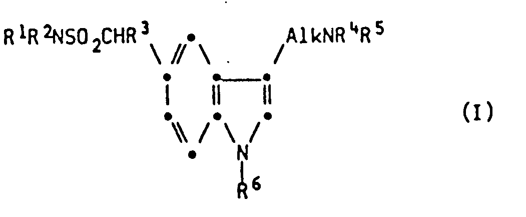 wherein R¹ represents a hydrogen atom or a C₁₋₆ alkyl or C₃₋₆ alkenyl group;
R² represents a hydrogen atom, a C₁₋₃ alkyl or C₃₋₆ alkenyl group, an aryl or ar(C₁₋₄)alkylene group, or a C₅₋₇ cycloalkyl group;
R³ represents a hydrogen atom or a C₁ -₃ alkyl group;
R⁴ and R⁵ which may be the same or different, each represents a hydrogen atom or a C₁ -₃ alkyl or 2-propenyl group, or R⁴ and R⁵ together form an aralkylidene group;
R⁶ represents a group -C0₂R⁷, COR⁷, -COC0₂R⁷, or -CONHR⁷, where R⁷ represents a hydrogen atom, a C, -4 alkyl group, a C₃ -₇ cycloalkyl group, a C₂-₄ alkenyl group, or an aryl or ar(Ci -₄)alkylene group (with the provisos that (a) R⁷ does not represent a hydrogen atom or a benzyl group when R⁶ is the group -C0₂R⁷ and (b) R⁷ does not represent an alkenyl group when R⁶ is the group -CONHR⁷); and
Alk represents an alkylene chain containing two or three carbon atoms which may be unsubstituted or substituted by not more than two C₁ -₃ alkyl groups,
and physiologically acceptable salts and solvates thereof.

These compounds have potent and selective vasoconstrictor activity and are therefore useful for the treatment of migraine.

US-A-3,562,278 discloses 4-substituted piperazine compounds having the formula: 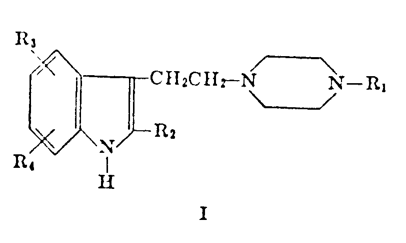 where R₁ is lower-alkyl, hydroxy-lower-alkyl, phenyl, phenyl-unbranched-lower-alkyl, cinnamyl, pyridyl, or 2- pyrimidyl; R₂ is carbo-lower-alkoxy, carboxy, sulfo-(SO₃H), hydroxymethyl, lower-alkanoyloxymethyl, carbamyl, N-lower-alkylcarbamyl, N,N-di-lower-alkylcarbamyl, formyl, or isonitrosomethylene (CH = NOH), and alkali metal salts of compounds where R₂ is carboxy or sulfo; R₃ is hydrogen, halogen, straight or unhindered lower-alkyl, lower-alkoxy, lower-alkylmercapto, trifluoromethyl, or hydroxy; and R₄ is hydrogen, halogen, straight or unhindered lower-alkyl or lower-alkoxy, or R₃ and R₄ together represent methylenedioxy or ethylenedioxy attached to adjacent carbon atoms, and wherein the benzene ring of R₁ as phenyl, phenyl-lower-alkyl, or phenyl-lower-alkenyl is unsubstituted or substituted by methylenedioxy or ethylenedioxy or by one or two of the same or different members of the group consisting of halogen, lower-alkyl, lower-alkoxy, lower-alkylmercapto, lower-alkylsulfinyl, lower-alkylsulfonyl, trifluoromethyl, or hydroxy.

These compounds possess psychomotor depressant activity and are therefore useful as tranquilizers.

Thus there is a need for a safe and effective drug for the treatment of migraine and related disorders which can be used either prophylactically or to alleviate an established headache.

The present invention relates to novel alkoxyprimidine derivatives, to their use in the treatment of vascular headaches, particularly migraine; to processes for their preparation and to their pharmaceutical compositions.

The compounds of the present invention are useful for the alleviation of vascular or vascular-related headache of which migraine and cluster are the best known specific examples. Their use involves administration to a human in need of such treatment. Oral and transnasal administration of pharmaceutical compositions containing the alkoxypyrimidine agent are preferred.

The alkoxypyrimidine derivatives having useful antimigraine serotonergic properties are represented by Formula I: 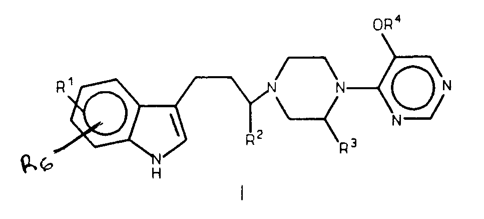 wherein
R¹ is selected from hydrogen, halogen, C1-C4 alkoxy, phenyl-C, -C4 alkoxy, hydroxy, and 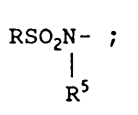
R², R³ and R⁵ are independently selected from hydrogen and C, -C₄ alkyl;
R⁴ is C₁-C₄ alkyl; R⁶ is hydrogen and halogen; or a pharmaceutically acceptable acid addition salt and/or solvate and/or stereoisomer thereof.

Additionally compounds of Formula I also encompass all pharmaceutically acceptable acid addition salts and/or solvates thereof. The present invention is also considered to include stereoisomers as well as optical isomers e.g. mixtures of enantiomers as well as individual enantiomers and diasteromers, which arise as a consequence of structural asymmetry in certain compounds of the instant series. Separation of the individual isomers is accomplished by application of various methods which are well known to practitioners in the art.

It is to be understood that, as used herein, halogen denotes fluorine, chlorine, bromine and iodine; with the term "lower alkyl" referring to both straight and branched chain carbon radicals of from 1 to 4 carbon atoms inclusive. Illustrative of these radicals are carbon chains which can be methyl, ethyl, propyl, isopropy, 1-butyl, 1-methylpropyl 2-methylpropyl.

The pharmaceutically acceptable acid addition salts of the invention are those in which the counter-ion does not contribute significantly to the toxicity or pharmacological activity of the salt and, as such, they are the pharmacological equivalents of the bases of Formula I. They are generally preferred for medical usage. In some instances, they have physical properties which makes them more desirable for pharmaceutical formulation such as solubility, lack of hygroscopicity, compressibility with respect to tablet formation and compatibility with other ingredients with which the substance may be used for pharmaceutical purposes. The salts are routinely made by admixture of a Formula I base with the selected acid preferably by contact in solution employing an excess of commonly used inert solvents such as water, ether, benzene, methanol, ethanol, ethyl acetate and acetonitrile. The may also be made by metathesis or treatment with an ion exchange resin under conditions in which the anion of one salt of the substance of the Formula I is replaced by another anion under conditions which allow for separation of the desired species such as by precipitation from solution or extraction into a solvent, or elution from or retention on an ion exchange resin. Pharmaceutically acceptable acids for the purposes of salt formation of the substances of Formula I include sulfuric, phosphoric, hydrochloric, hydrobromic, hydroidic, citric, acetic, benzoic, cinnamic, mandelic, phosphoric, nitric, mucic, isethionic, palmitic, heptanoic, and others.

The compounds of Formula I can be prepared by means of the processes shown in Scheme 1.

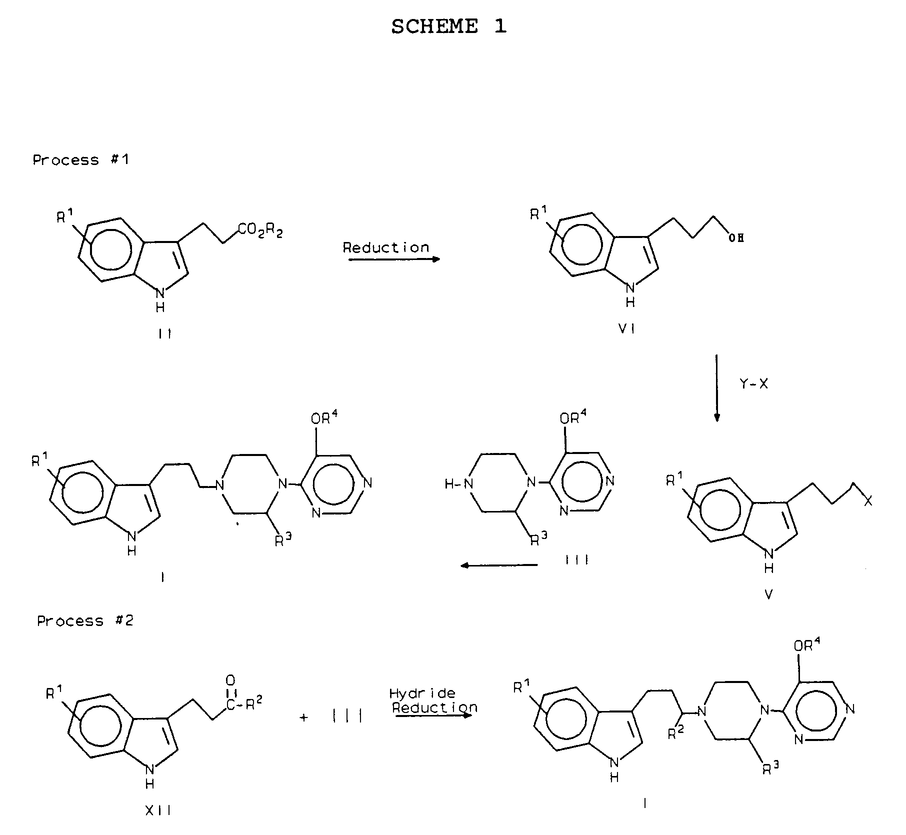

For the two processes of Scheme 1, R¹ through R⁴ are as defined hereinabove. The reagent Y-X represents an organic leaving group reagent wherein X is the leaving group fragment such as tosyl, mesyl, halide, sulfate, phosphate and so forth; and Y is either a proton or a counter ion: e.g. Y-X can be HBr or tosyl chloride and the like. "Hydride reduction" concerns intended reductive amination of XII by III, particularly the reduction of the initial complex of XII and III to provide product I. A preferred reagent for this use in Process #2 is NaCNBH₃. The reagents of Scheme I and their acronyms are familiar to the practitioner skilled in organic synthesis and their structure and usage would be readily understood.

Process #1 in Scheme 1 comprises the reduction of an indole carboxylic acid or ester of formula II to the corresponding alcohol of formula VI which is converted to an activated intermediate of formula V in which the alcoholic moiety is now an organic leaving group. Reaction of intermediate V with a pyrimidinylpiperazine of formula III then provides product I.

Process #2 comprises combination of the indole intermediate of formula XII with a pyrimidinylpiperazine intermediate of formula III followed by treatment with sodium cyanoborohydride to give the product of formula I.

Reagents, solvents, and reaction conditions for the above described steps of the two processes would be known to one skilled in organic synthesis as all the steps comprise standard organic reactions, the details of which are readily available in the chemical literature. These processes may be adapted to variation in order to produce other compounds embraced by this invention but not specifically disclosed. Variations of the methods to produce the same compounds in somewhat different fashion will also be evident to one skilled in the art.

To provide greater descriptive detail, representative synthetic examples are provided hereinbelow in the "Description of Specific Embodiments" section.

Serotonin has been linked to the pathophysiology of migraine by accumulating evidence including increased excretion of serotonin metabolites following a migraine attack and a reduction in the serotonin content of blood platelets during the migraine headache. This latter effect appears to be specific for migraine and not a result of pain or stress. (Anthony, et al., "Plasma serotonin in migraine and stress", Arch. Neurol. 1967, 16: 544-552). More importantly, intramuscular injection of reserpine lowers plasma serotonin and induces a typical migraine-type headache in migraine sufferers. This induced headache can be alleviated by slow I.V. injection of serotonin creatinine sulfate. (Kimball, et al., "Effect of serotonin in migraine patients", Neurology N.Y., 1960, 10: 107-111).

Although serotonin has been shown to be effective in treating migraine attacks, its use in migraine is precluded by its side-effects such as restlessness, nausea, faintness, hyperpnea, facial flushing and parasthesias. (Lance, et al., "The control of cranial arteries by humoral mechanisms and its relation to the migraine syndrome", Headache, 1967, 7: 93-102). For this reason more specific serotonin agents, which would treat the migraine without all of the other actions, are potentially useful antimigraine medicaments. Accumulating findings have led to the perception that compounds with selectivity for the 5-HTᵢₒ sub-type of serotonin receptors would be clinically efficacious in the treatment of migraine. In this regard the compounds of the instant invention demonstrate potent affinity and agonist activity at the 5-HTᵢₒ site. The high potencies of these compounds is shown by IC₅₀ values being below 5 nmolar.

Determination of 5-HTᵢₒ binding properties was accomplished employing methodology such as that described by Heuring and Peroutka, J. Neurosci., 7(3), 1987, 894-903; with only minor modifications. In vitro IC₅₀ (nM) test values were determined for the compounds of this invention employing tritiated serotonin.

Another aspect of the instant invention provides a method for treating a migraine sufferer which comprises systemic administration to the sufferer of a therapeutically effective amount of a compound of Formula I or a pharmaceutically acceptable salt thereof.

The administration and dosage regimen of compounds of Formula I is considered to be done in the same manner as for the reference compound sumatriptan, cf: Oxford, GB 2,162,522A. Although the dosage and dosage regimen must in each case be carefully adjusted, utilizing sound professional judgment and considering the age, weight and condition of the recipient, the route of administration and the nature and gravity of the illness, generally the daily dose will be from about 0.05 to about 10 mg/kg, preferably 0.1 to 2 mg/kg, when administered parenterally and from about 1 to about 50 mg/kg, preferably about 5 to 20 mg/kg, when administered orally. In some instances, a sufficient therapeutic effect can be obtained at lower doses while in others, larger doses will be required. Systemic administration refers to oral, intra-nasal, rectal and parenteral (i.e. intramuscular, intravenous and subcutaneous). Generally, it will be found that when a compound of the present invention is administered orally, a larger quantity of the active agent is required to produce the same effect as a smaller quantity given intra-nasally or parenterally. In accordance with good clinical practice, it is preferred to administer the instant compounds at a concentration level that will produce effective antimigraine effects without causing any harmful or untoward side effects.

The compounds of the present invention may be administered for antimigraine purposes either as individual therapeutic agents or as mixtures with other therapeutic agents. Therapeutically, they are generally given as pharmaceutical compositions comprised of an antimigraine amount of a compound of Formula I or a pharmaceutically acceptable salt thereof and a pharmaceutically acceptable carrier, excipient or diluent thereof. Pharmaceutical compositions which provide from about 1 to 500 mg of the active ingredient per unit dose are preferred and are conventionally prepared as tablets, lozenges, capsules, powders, aqueous or oily suspensions, syrups, elixirs, and aqueous solutions.

The nature of the pharmaceutical composition employed will, of course, depend on the desired route of administration. For example, oral compositions may be in the form of tablets or capsules and may contain conventional excipients such as binding agents (e.g. starch) and wetting agents (e.g. sodium lauryl sulfate). Solutions or suspensions of a Formula I compound with conventional pharmaceutical vehicles are employed for intra-nasal and parenteral compositions such as an aqueous solution for intravenous injection or an oily suspension for intramuscular injection.

The compounds which constitute this invention, their methods of preparation and their biologic actions will appear more fully from consideration of the following examples, which are given for the purpose of illustration only and are not be construed as limiting the invention in sphere or scope. In the following examples, used to illustrate the foregoing synthetic processes, temperatures are expressed in degrees Celsius and melting points are uncorrected. The nuclear magnetic resonances (NMR) spectral characteristics refer to chemical shifts (6) expressed as parts per million (ppm) versus tetramethylsilane (TMS) as reference standard. The relative area reported for the various shifts in the 'H NMR spectral data corresponds to the number of hydrogen atoms of a particular functional type in the molecule. The nature of the shifts as to multiplicity is reported as broad singlet (bs), singlet (s), multiplet (m), triplet (t) or doublet (d). Abbreviations employed are DMSO-d₆ (deuterodimethylsulfoxide), CDCI₃ (deuterochloroform) and are otherwise conventional. The infrared (IR) spectral descriptions include only absorption wave numbers (cm-¹) having functional group identification value. The IR determinations were employed using potassium bromide (KBr) as diluent. The elemental analyses are reported as percent by weight.

The following examples describe in detail the preparation of compounds of Formula I, as well as synthetic intermediates in each process. It will be apparent to those skilled in the art that modifications, both of materials and methods, will allow preparation of other compounds disclosed herein. From the foregoing description and the following examples it is believed that one skilled in the art is able to use the invention to the fullest extent.

### A. Preparation of Intermediate Compounds

Some representative procedures for preparation of synthetic intermediate compounds utilized in the three processes of Scheme 1 are given hereinbelow. Most starting materials and certain intermediates (e.g. Formula II and V compounds), are either commercially available or procedures for their synthesis are readily available in the chemical literature allowing their full utilization by one skilled in the art of organic synthetic chemistry.

### Compounds of Formula 11

### Example 1

### General Method: 5-Fluoroindole-3-propionic acid

A modification of a procedure reported by Johnson¹ for the preparation of indole-3-propionic acid was used.

Thus, a solution of 5-fluoroindole (1.35 g, 0.010 mol) in 10 mL of acetic acid containing acrylic acid (1.5 mL, 0.022 mol) and acetic anhydride (1.9 mL, 0.02 mol) was heated (oil bath) at 90 _{°} C under Ar for 5 days. The volatiles were then removed in vacuo and the residue was taken up in 3N NaOH. Insoluble material was removed by filtration and the filtrate was acidified with conc. HCI and then extracted with CH₂CI₂. The organic extract was dried (Na₂SO₄) and evaporated to give the product (1.191 g, 57%) as a solid which was used without further purification: IR (neat) 3420, 1710 cm-¹; ¹Hnmr (200 MHz, CDCI₃) δ 7.94 (br s, 1H), 7.28-7.18 (m, 3H), 7.05 (d, J = 2.5 Hz, 1H), 6.93 (dt, J=9.0, 2.6 Hz, 1H), 3.05 (t, J=7.5 Hz, 2H), 2.73 (t, J = 7.6 Hz, 2H).

By appropriate modification of the general method, other Formula II compounds are readily obtainable.

### Example 2

### 5-Chloroindole-3-propionic acid

The crude product was chromatographed (Si0₂/5-20% ethyl acetate-CH₂CI₂) to give the title compound as a beige solid, m.p. 100-102 ° C: Yield=41 %; IR (neat) 3435, 1695 cm⁻¹; ¹Hnmr (200 MHz, CDCI₃) 6 8.00 (br s, 1 H), 7.56 (d, J = 1.8 Hz, 1 H), 7.27 (d, J = 8.7 Hz, 1 H), 7.15 (dd, J = 8.6, 1.9 Hz, 1 H), 7.05 (d, J = 2.3 Hz, 1 H), 3.07 (t, J = 7.5 Hz, 2H), 2.75 (t, J = 7.4 Hz, 2H).

### Example 3

### 6-Fluoroindole-3-propionic acid

The crude product was chromatographed (Si0₂/20% hexane-ethyl acetate) to give the title compound as a beige solid, m.p. 98-102°C: Yield=23%, IR (neat) 3400, 1687 cm⁻¹; ¹Hnmr (200 MHz, CDCI₃) 6 7.97 (br s, 1 H), 7.50 (dd, J = 8.7, 5.4 Hz, 1 H), 7.07-6.99 (m, 2H), 6.89 (dt, J = 9.5, 2.2 Hz, 1 H), 3.09 (t, J = 7.5 Hz, 2H), 2.76 (t, J = 7.4 Hz, 2H).

### 'H.E. Johnson and D.G. Crosby, J. Org. Chem., 25, 569 (1969)

### Compounds of Formula 11

### Example 4

### 3-[5-(Methylsulfonyl)aminoindolyl]propanoic acid

### A. 5-(Methylsulfonyl)aminoindole

To a solution of 20 g of 5-aminoindole (151 mmol, 1.0 equiv.) and 31.5 mL (226 mmol, 1.5 equiv.) of triethylamine in 200 mL of CH₂CI₂ at 0 ° C was added a solution of 12.8 mL (166 mmol, 1.1 equiv.) of methanesulfonyl chloride in 50 mL of CH₂CI₂ dropwise. The solution was allowed to slowly warm to room temperature over 16 hours. The reaction mixture was concentrated and the residue dissolved in 800 mL of ethyl acetate. The organic layer was washed with 200 mL of water, 100 mL of 0.1 M HCI, 100 mL of saturated aqueous sodium bicarbonate and 100 mL of saturated aqueous sodium chloride. The organic layer was dried over anhydrous sodium sulfate, filtered, and concentrated in vacuo to obtain 31.55 g (100%) of 5-methanesulfonamidoindole, m.p. 133-135 ° C.

### B. 3-[5-Methylsulfonyl)aminoindolyl]propanoic acid

To a solution of 3.4 g (16.2 mmol, 1.0 equiv.) of 5-methanesulfonamidoindole in 15 mL of glacial acetic acid was added 2.2 mL (32.3 mmol, 2.0 equiv.) of acrylic acid and 3.1 mL (32.3 mmol, 2.0 equiv.) of acetic anhydride. The reaction mixture was heated at 90 _{°} C for 18 hours. The reaction mixture was cooled and volatile material was removed in vacuo. The residue was dissolved in 1 M sodium hydroxide with heating. Insoluble material was removed by filtration. The basic aqueous layer was acidified to pH 1 with concentrated aqueous hydrochloric acid. The aqueous layer was extracted with three 50 mL portions of ethyl acetate. The combined organic layers were dried over sodium sulfate, filtered and concentrated to give 5.0 g of a brown oil whose ¹HNMR and mass spectrum where consistent with formation of the carboxylic acid II. The material was used without further purification in subsequent reactions.

### Compounds of Formula VI

### Example 5

### General Method: 5-Fluoro-3-(3-hydroxypropyl)indole

To a suspension of LiA1H₄ (433 mg, 11.4 mmol) in 20 mL of dry tetrahydrofuran at 5-10 ° C under Ar was added a solution of 5-fluoroindole-3-propionic acid (1.179 g, 5.7 mmol) in 5 mL of tetrahydrofuran. After 10 min the cooling bath was removed and the mixture was stirred at room temperature for 30 min and finally it was heated to reflux for 30 min. The resulting gummy mixture was allowed to cool to room temperature and then the reaction was quenched by the sequential addition of 0.5 mL of H₂0, 0.5 mL of 15% NaOH solution and finally 1.5 mL of H₂0. The mixture was then diluted with ethyl acetate, dried (MgS0₄) and evaporated to give a yellow-green oil. Flash chromatography (SiO₂/CH₂CI₂-ethyl acetate=2:1) afforded the product (918 mg, 83%) as an oil: IR (neat) 3420, 1583 cm⁻¹; ¹Hnmr (200 MHz, CDCI₃) δ 7.94 (br s, 1 H), 7.28-7.20 (m, 2H), 7.03 (d, J = 2.4 Hz, 1 H), 6.92 (dt, J = 9.1, 2.5 Hz, 1 H), 3.71 (t, J = 6.4 Hz, 2H), 2.80 (t, J = 7.5 Hz, 2H), 2.02-1.88 (m, 2H), 1.33 (br s, 1 H).

### Example 6

### 5-Chloro-3-(3-hydroxypropyl)indole

The title compound was prepared according to the general procedure to give a light brown oil which was used without further purification: Yield=96%; IR (neat) 3430, 3300, 1462 cm-¹.

### Example 7

### 6-Fluoro-3-(3-hydroxypropyl)indole

The product was prepared according to the general procedure, except that the reaction was run at room temperature for 5 h. Standard work-up gave the title compound as a light brown gum: Yield=90%; IR (neat) 3420, 1630 cm⁻¹.

### Example 8

### 3-[5-(Methylsulfonyl)aminoindolyl]propanol

To a solution of 2.02 g (7.16 mmol, 1.0 equiv.) of the crude indolepropionic acid (Example 4) in 10 mL of THF at 0 ° C was added 32.2 mL of a 1 M solution of borane-THF complex in THF dropwise. After the addition was complete, the ice bath was removed and the reaction mixture was allowed to warm to room temperature over thirty minutes. The reaction mixture was poured into saturated aqueous sodium bicarbonate and extracted with three 25 mL portions of ethyl acetate. The combined organic layers were dried over anhydrous sodium sulfate, filtered and concentrated in vacuo to yield 1.82 g of a yellow oil. This was chromatographed on silica gel using 5% methanol in methylene chloride containing 0.5% concentrated aqueous ammonia to yield 0.900 g (47% overall from 5-methanesulfonamidoindole) of VI.

### Compounds of Formula V

### Example 9

### General Method: 5-Fluoro-3-(3-p-toluenesulfonyl oxypropyl)indole

To a solution of 5-fluoro-3-(3-hydroxypropyl)indole (917 mg, 4.75 mmol) in 20 mL of CH₂CI₂ at 0 ° C under Ar was added triethylamine (728 µL, 5.23 mmol), followed by a solution of p-toluenesulfonyl chloride (994 mg, 5.23 mmol) in 5 mL of CH₂CI₂ and then a catalytic amount of 4-dimethylaminopyridine (59 mg, 0.48 mmol). The reaction mixture was stirred at 0 ° C for 30 min and then at room temperature for 1 1/2 h. Evaporation of the mixture followed by chromatography (Si0₂/CH₂CI₂) of the residue gave a gum. The gum was dissolved in ether and then the solution was diluted with hexane until an oil separated. Addition of a small amount of CH₂ CI₂ led to dissolution of the oil and crystallization of the product. Storage at - 20 ° C and then filtration and drying of the residue in vacuo gave the product (1.378 g, 84%) as fluffy white needles: m.p. 99 ° C; IR (CH₂CI₂) 3470, 1360, 1178 cm⁻¹; ¹Hnmr (200 MHz, CDCIa) δ 7.90 (br s, 1H), 7.61 (d, J=8.4 Hz, 2H), 7.30 (d, J=8.4 Hz, 2H), 7.27-7.20 (m, 1 H), 7.08 (dd, J=9.6, 2.6 Hz, 1 H), 6.96-6.94 (m, 1 H), 6.88 (dd, J = 9.0, 2.5 Hz, 1 H), 4.06 (t, J = 6.2 Hz, 2H), 2.74 (t, J = 7.2 Hz, 2H), 2.42 (s, 3H), 1.99 (dq, J = 7.2, 6.2 Hz, 2H).

### Example 10

### 5-Chloro-3-(3-p-toluenesulfonyloxypropyl)indole

The crude product, produced via appropriate modification of Example 9, was chromatographed (Si0₂/ethyl acetate-hexane = 1:1) to give the title compound as a solid, m.p. 80-83 _{°} C: Yield = 80%; IR (neat) 3442, 1350, 1175 cm-¹.

### Example 11

### 6-Fluoro-3-(3-p-toluenesulfonyloxypropyl)indole

The crude product, produced via appropriate modification of Example 9 was chromatographed (Si0₂/10-30% ethyl acetate-hexane) to give the title compound as an oil: Yield=84%; IR (neat) 3410, 1353, 1178 cm⁻¹.

### Example 12

### 3-(3-bromopropyl)-1 H-indole

Phosphorus tribromide (17.4 g) in Et₂0 (30 mL) was added dropwise to a Et₂0 solution (100 mL) containing 3-(3-hydroxypropyl)indole (VI; 7.5 g) at 0 ° C with stirring and under N₂ atmosphere. After the addition was complete, the reaction was allowed to warm to 23 ° C and continuously stirred for 16 h. At the end of this time, the reaction was cooled to 0 ° C and ice (ca. 25 mL) added portionwise and stirred an additional 2 h. The organic phase was separated from the aqueous phase and the aqueous layer extracted with Et₂0. The combined organic phases were washed with sat. NaCl solution, dried with MgS0₄, filtered, and concentrated under reduced pressure to afford 3-(3-bromopropyl)indole (V; 1.51 g; 15%).

### Example 13

### 3-[5-(Methylsulfonyl)aminoindolyl]propanol methanesulfonate

To a solution of 0.0920 g (0.343 mmol, 1.0 equiv.) of the indolepropanol in 3.5 mL of THF at 0 ° C was added 0.10 mL (0.686 mmol, 2.0 equiv.) of triethylamine followed by 0.04 mL of methanesulfonyl chloride. The reaction mixture was stirred at 0 ° C for thirty minutes then poured into 15 mL of saturated aqueous sodium bicarbonate. The aqueous layer was extracted with two five mL portions of ethyl acetate. The combined organic layers were dried over anhydrous sodium sulfate, filtered and concentrated in vacuo to yield 0.127 g of a yellow oil whose ¹HNMR indicated formation of the mesylate V.

### Compounds of Formula III

### Example 14

### 1-(5-Methoxy-4-pyrimidinyl)piperazine -- Method 1

To a solution of piperazine (38.40 g, 0.45 mole) in CH₃CN (225 mL) was added dropwise a CH₃CN (100 mL) solution containing 4-chloro-5-methoxypyrimidine (6.45 g, 0.04 mole) while under nitrogen atmosphere. After the addition was complete the reaction was heated at 60 ° C for 0.75 h. The reaction was concentrated under reduced pressure and the residue dissolved in CH₂CI₂ and extracted with 5% NaHC0₃ and H₂0. The organic phase was dried with K₂C0₃, filtered, and concentrated under reduced pressure. Silica gel chromatography (CH₂CI₂: MeOH:NH₄0H; 92:8:0.8) of the concentrate afforded II (7.63 g, 88.1%). Treatment of the base (1.0 g) with ethanolic HCI and crystallization from EtOH/i-PrOH yielded the hydrochloride salt of II (0.50 g, 39.1%, m.p. 207-211 °).

### 1-(5-Methoxy-4-pyrimidinyl)piperazine -- Method 2

### A. 4,6-Dihydroxy-5-methoxypyrimidine

A modified procedure of Bretschneider, Richter, and Klötzer, Monatsh. Chem. 96(6), 1661-76 (1965), was used. Absolute methanol (1.0 L) was added with ice bath cooling to sodium methoxide (175 g, 3.24 moles) in a 3 L round bottom flask. When the mixture had cooled to less than 20 ° C, dimethyl methoxymalonate (162.14 g, 1.00 mole) was added, and then solid formamidine acetate (104.11 g, 1.00 mole) was added. The mixture was stirred in the ice bath for 30 minutes, and then refluxed for 1 hour. The mixture was cooled in a cold water bath and then concentrated HCI (about 350 mL) was added until the mixture was strongly acidic on pH test paper. The precipitate was filtered, suspended in cold water (about 400 mL), and then filtered again. The white powder was dried in vacuo (125.84 g, 88.7%), and carried on without further purification.

### B. 4,6-Dichloro-5-methoxypyrimidine

A modified procedure of Bretschneider, Richter, and Klötze, Monatsh. Chem. 96(6), 1661-76 (1965), was used. A mixture of 4,6-dihydroxy-5-methoxy-pyrimidine (125.84 g, 0.887 mole), POCI₃ (700 mL), and N,N-diethylaniline (50 mL) was refluxed for 3 hours to give a brown solution. The solution was cooled and then the excess POCI₃ was removed in vacuo. Hexane (about 300 mL) was added to the residue and the mixture was refluxed with stirring. The hot hexane layer was decanted into a beaker, and residue treated two more times with hot hexane. The hexane extracts (total volume about 1 L) were concentrated in vacuo to give the crude product as a white solid (116.5 g, 73.3%). This material was recrystallized from pet ether to give colorless needles (92.0 g + 16.51 g second crop, 93.1% total recovery).

### C. 6-Chloro-5-methoxy-4-(1-piperazinyl)pyrimidine

Piperazine (30 g) was dissolved in water (150 mL) and then solid 4,6-Dichloro-5-methoxypyrimidine (10.00 g, 55.8 mmole) was added. The mixture was vigorously stirred for 2 h at room temperature during which the 4,6-dichloro-5-methoxypyrimidine dissolved. The product was extracted from the aqueous reaction mixture with methylene chloride (yield 12.67 g, 99.2%). A sample (5 g) of the crude product was chromatographed on silica gel using a gradient of 20-40% methanol/ethyl acetate as the eluent. The product was then dissolved in acetonitrile and concentrated HCI added to give the salt as a white powder which was dried in vacuo to give the analytical sample (4.0 g, m.p.: 169-173 ° C bubbles).

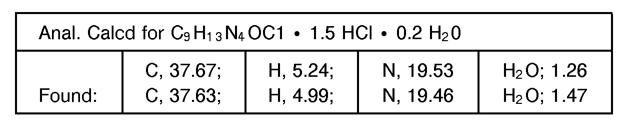

### D. 1-(5-Methoxy-4-pyrimidinyl)piperazine

Piperazine (20 g) was dissolved in water (100 mL) in a Parr bottle and then solid 4,6-dichloro-5-methoxypyrimidine (5.00 g, 27.9 mmole) was added. The mixture was vigorously stirred for 2 h at room temperature during which the 4,6-dichloro-5-methoxypyrimidine dissolved. The stirring bar was removed, catalyst (10% Pd/C, 1.0 g) was added to the turbid solution, and the mixture was then hydrogenated (60 psi, 3 h) at room temperature. The catalyst was filtered off and the filtrate extracted 3 times with CH₂CI₂. The CH₂CI₂ extracts were dried over Na₂SO₄ and concentrated in vacuo to give a clear oil which solidified upon standing (3.34 g, 61.7%). This crude product was Kugelrohr distilled (yield 3.24 g), dissolved in acetonitrile, and concentrated HCI was added to precipitate the product as a white powder which was dried in vacuo - (4.32 g, 94.0% from crude product, m.p. 219-221.5 ° C).

### Example 15

### 1-(5-Methoxy-4-pyrimidinyl)-2-methylpiperazine --Method 1

### A. 1-(t-Butoxycarbonyl)-3-methylpiperazine

To a cold (-5 ° C) solution of 2-methylpiperazine (5.00 g, 0.05 mole) in 200 mL of CH2CI2 under Ar was added a solution of di-t-butyl dicarbonate (10.9 g, 0.05 mole) in 100 mL of CH₂CI₂ over 1 h. The resulting mixture was stirred at -5 ° C for 1 h and then at r.t. for 2 h. The solution was then washed (H20), dried (Na₂S0₄) and evaporated to give an oil which was chromatographed (Si0₂/ethyl acetate then ethyl acetate-MeOH-NH₄OH= 10:1:0.1) to give the product (4.30 g, 43%) as an oil. This material was used without further purification: ¹H nmr (200 MHz, CDCIa) δ 4.15-3.75 (br s, 2H), 3.0-2.6 (m, 4H), 2.47-2.35 (m, 1H), 1.48 (s, 9H), 1.08 (d, J = 6.7 Hz, 3H).

### B. 1-(t-Butoxycarbonyl)-4-(5-methoxy-4-pyrimidyl)-3-methylpiperazine

A mixture of 1-(t-butoxycarbonyl)-3-methylpiperazine (2.0 g, 0.01 mole), 4-chloro-5-methoxypyrimidine (1.5 g, 0.01 mole) and diisopropylethylamine (2.6 mL, 0.015 mole) in 25 mL of dry acetonitrile was heated to reflux under Ar for 60 h. The resulting solution was diluted with ether and then washed (H₂0, brine), dried (Na₂S0₄) and evaporated to give a gum. This gum was triturated with hexane (x3) and the supernatant was evaporated to give a gum. Flash chromatography (Si0₂/ethyl acetate-hexane=1:1, then ethyl acetate) of this material gave first 4-chloro-5-methoxypyrimidine (0.4 g, 27%) and then the desired product (1.2 g, 30%) as a light pink solid: m.p. 70-72 ° C; IR (KBr) 1690, 1575 cm⁻¹; ¹H nmr (200 MHz, CDCI₃) δ 8.33 (s, 1 H), 7.90 (s, 1 H), 4.79 (br s, 1 H), 4.4-3.8 (m, 3H), 3.86 (s, 3H), 3.35-2.90 (m, 3H), 1.48 (s, 9H), 1.21 (d, J = 6.7 Hz, 3H).

### C. 1-(5-Methoxy-4-pyrimidinyl)-2-methylpiperazine

A solution of 1-(t-butoxycarbonyl)-4-(5-methoxy-4-pyrimidinyl)-3-methylpiperazine (1.70 g, 4.2 mmol) and trifluoroacetic acid (5 mL) in 50 mL of CH₂CI₂ was stirred at r.t. under Ar for 18 h. The solution was evaporated, the residue was taken up in water and the mixture was basified (pH8) with 15% aqueous NaOH. The resulting (pH 8) mixture was extracted with ethyl acetate and the organic phase was washed (H₂0, brine), dried (Na₂S0₄) and evaporated to give a semi-solid. This material was taken up in ether, filtered to remove insoluble material and the solution was evaporated to give the product (0.80 g, 92%) as an oil. It was used without further purification: IR (neat) 3300, 1580, 1540 cm⁻¹; ¹H nmr (200 MHz, CDCI₃) δ 8.32 (s, 1 H), 7.87 (s, 1 H), 4.83-4.68 (m, 1 H), 4.26-4.19 (m, 1 H), 3.85 (s, 3H), 3.26-3.17 (m, 1H), 3.12-3.01 (m, 2H), 2.94-2.80 (m, 2H), 1.29 (d, J=6.8 Hz, 3H).

### 4-(5-Methoxy-4-pyrimidinyl)-2-methylpiperazine --Method 2

A solution of 2-methylpiperazine (20 g) in water (100 mL) was reacted with solid 4,6-dichloro-5-methoxypyrimidine (5.00 g, 27.9 mmole) in a procedure similar to that given for Method 2 of Example 14. After hydrogenation and filtration of the catalyst, the product was extracted from the filtrate with CH₂CI₂. The extracts were concentrated in vacuo, and the residue was Kügelrohr distilled to give a clear oil (5.46 g, 99.8%). The oil was dissolved in acetonitrile and concentrated HCI added to form the salt which was recrystallized from i-PrOH and dried in vacuo to give the product as a white powder (4.02 g, m.p. 185-188 ° C).

### Example 16

### 1-(5-Ethoxy-4-pyrimidinyl)piperazine

### A. 5-Ethoxypyrimidine

Sodium (2.89 g. 125.8 mmol) was dissolved in ethanol (110 mL) and 5-bromopyrimidine (10.0 g, 62.9 mmol) added. The reaction was heated at 120 ° in an autoclave for 17 h and then allowed to stand at 23 for 60 h. The ethanol was removed under reduced pressure and water (5 mL) added to the concentrate. The aqueous phase was extracted with CH₂CI₂ (4 x 100 mL). The combined organic extracts were washed with saturated NaCl solution, dried with anhydrous K₂C0₃, filtered, and concentrated under reduced pressure. Silica gel chromatography (Hexane/EtOAc; 70:30) of the concentrate yielded 5-ethoxypyrimidine (2.00 g, 25.6%).

### B. 5-Ethoxypyrimidine N-oxide

To a solution of 5-ethoxypyrimidine (2.00 g, 16.1 mmol) in CH₂CI₂ (100 mL) was added 3-chloroperoxybenzoic acid, 50-60% tech. grade, (6.13 g, 17.7 mmol) and the reaction stirred at 23 ° C for 18 h. The reaction was extracted with water (2 mL) containing Na₂C0₃ (1.71 g, 16.1 mmol). The organic phase was dried with anhydrous K₂C0₃, filtered, and concentrated under reduced pressure to afford the N-oxide (1.75 g, 78%).

### C. 4-Chloro-5-ethoxypyrimidine

To a solution of triethylamine (1.90 g, 18.6 mmol) and phosphorous oxychloride (2.87 g, 18.6 mmol) in CHCI₃ (60 mL) was added 5-ethoxypyrimidine N-oxide (1.75 g, 12.5 mmol) portionwise. After the addition was complete, the reaction was heated at reflux for 3 h. Upon cooling the mixture to 0 ° C, CHCI₃ (60 mL) and water (10 mL) were added following by portionwise addition of NaHC0₃ (3.15 g, 37.5 mmol). After the effervescence had ceased, the organic phase was separated, dried with MgS0₄, filtered, and concentrated under reduced pressure to afford the chloro product (1.98 g) which was used without further purification.

### D. 4-(Ethoxycarbonyl)-1-(5-ethoxy-4-pyrimidinyl) piperazine

A mixture of 4-chloro-5-ethoxypyrimidine (1.98 g, 12.5 mmol), ethyl 1-piperazinecarboxylate (5.93 g, 37.5 mmol) and micropulverized K₂C0₃ (5.18 g, 37.5 mmol) was heated at reflux in CH₃CN (75 mL) for 4 h. Upon cooling to 23 ° C, the solvent was removed under reduced pressure. The residue was dissolved in CH₂CI₂ and washed with water (5 mL). The organic phase was dried with anhydrous K₂CO₃,filtered, and concentrated under reduced pressure. Silica gel chromatography (CH₂CI₂/MeOH;98:2) of the concentrate yielded product (2.29 g, 65%).

### E. 1-(5-Ethoxy-4-pyrimidinyl)piperazine

To a KOH solution (10N, 20 mL) was added 4-(ethoxycarbonyl)-1-(5-ethoxy-4-pyrimidinyl)piperazine (2.29 g, 8.18 mmol). The reaction was heated at reflux for 24 h after which time the water was removed under reduced pressure. The residue was dissolved in CH₂CI₂, washed with saturated NaCl solution, dried with anhydrous K₂C0₃, filtered, and concentrated under reduced pressure. Silica gel chromatography (CH₂CI₂/MeOH/NH₄0H;95:5:0.5) of the concentrate yielded III (0.71 g, 42%).

### Products of Formula I

### Example 17

### 1-[3-(5-Fluoro-1H-indol-3-yl)propyl]-4-(5-methoxy-4-pyrimidinyl)piperazine

To a solution of 5-fluoro-3-(3-p-toluenesulfonyloxy-propyl)-indole (1.16 g, 3.34 mmol) in 50 mL of acetonitrile was added 1-(5-methoxy-4-pyrimidyl) piperazine (0.78 g, 4.0 mmol), KI (0.56 g, 4.0 mmol) and diisopropylethylamine (3.48 mL, 20.0 mmol) and the mixture was heated to reflux under Ar for 20 h. The resulting mixture was diluted with ethyl acetate, washed (H₂0, brine), dried (Na₂S0₄) and evaporated to give a gum. Flash chromatography (Si0₂/ethyl acetate-methanol 95:5) of this material gave a gum which was triturated with CH₂CI₂-ether. Evaporation of the supernatant gave a foam which was recrystallised from ethyl acetate-hexane to give the product (0.70 g, 57%) as off-white crystals: m.p. 119-122_{°}C; IR (KBr) 3190, 1580 cm⁻¹; ¹Hnmr (200 MHz, CDCI₃) δ 8.33 (s, 1H), 8.04 (br 2, 1H), 7.89 (s, 1H), 7.29-7.21 (m, 2H), 7.04 (d, J = 2.2 Hz, 1 H), 6.93 (dt, J = 9.1, 2.4 Hz, 1 H), 3.85 (s, 3H), 3.80 (t, J = 5 Hz, 4H), 2.76 (t, J = 7.5 Hz, 2H), 2.55 (t, J=5 5 Hz, 4H), 2.50-2.43 (m, 2H), 2.00-1.85 (m, 2H).

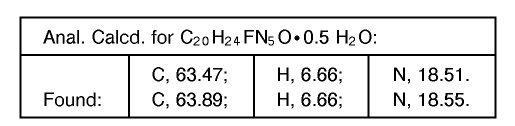

### Example 18

### 4-(5-Ethoxy-4-pyrimidinyl)-1-[3-(1 H-indol-3-yl)propyl]piperazine hydrochloride hydrate

A mixture of 3-(1H-indole-3-yl)propyl 4-methylbenzenesulfonate (0.87 g, 2.65 mmol), 1-(5-ethoxy-4-pyrimidinyl)piperazine (1.10 g, 5.29 mmol), micropulverized K₂C0₃ (0.73 g, 5.29 mmol), and tetrabutylammonium hydrogen sulfate (0.04 g, 0.13 mmol) in CH₃CN (15 mL) was heated at reflux for 2.5 h under nitrogen atmosphere. The reaction was allowed to cool to 23 ° C and stand for 16 h. The reaction was concentrated under reduced pressure and the residue was dissolved in CH₂CI₂ and extracted with water. The organic phase was dried with anhydrous K₂C0₃, filtered, and concentrated under reduced pressure. Silica gel chromatography (CH₂CI₂/MeOH;98:2) of the residue afforded the free base (0.70 g, 72%) which was treated with ethanolic HCI to yield I (0.85 g, 99%; mp >230 ° C) after crystallization from EtOH/MeOH.

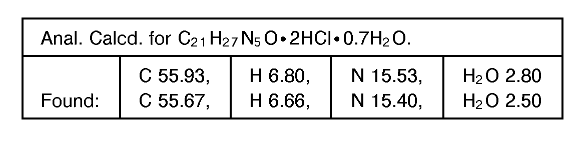

### Example 19

### 1-[3-(1 H-Indol-3-yl)propyl]-4-(5-methoxy-4-pyrimidinyl)piperazine hydrochloride

A mixture of 3-(1H-indol-3-yl)propyl 4-methylbenzenesulfonate (3.39 g, 10.31 mmol), 1-(5-methoxy-4-pyrimidinyl)-piperazine (2.0 g, 10.31 mmol), N,N-diisopropylethylamine (1.33 g, 10.31 mmol), and potassium iodide (0.26 g, 1.55 mmol) in dimethylformamide (6 mL) was heated at 50 ° C for 18 h under nitrogen atmosphere. The reaction was concentrated under reduced pressure. Silica gel chromatography (EtOAc:Et₃N:MeOH; 92:4:4) of the concentrate yielded the free base (2.35 g, 64.9%) which was treated with ethanolic HCI. Recrystallization from isopropanol afforded compound I (2.37 g, 57.5%; m.p. 221-224 C°).

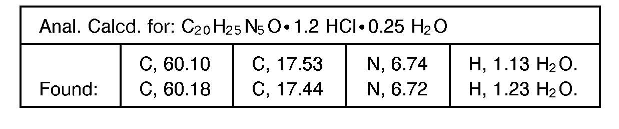

### Example 20

### 1-[3-(1 H-indol-3-yl)butyl]-4-(5-methoxy-4-pyrimidinyl)piperazine

To a mixture of 1-(5-methoxy-4-pyrimidyl)piperazine (1.55 g, 8.0 mmol), triethylamine hydrochloride (1.10 g, 8.0 mmol) and NaCNBH₃ (1.76 g, 28 mmol) in 20 mL of dry THF was added a solution of 3-(3-oxobutyl)indole², (0.75 g, 4.0 mmol) in 5 mL of THF and the mixture was stirred at room temperature under Ar for 20 h. The reaction mixture was then poured into 10% saturated NaHC0₃ and extracted with ethyl acetate (x2). The organic extract was washed with H₂O (x2) and then with 25 mL of 0.1 N HCI. The resulting organic solution was extracted with 1N HCI (x2) and the aqueous phase was washed with CH₂CI₂ (x2) and then it was cooled at 0 ° C and basified with 50% aqueous NaOH. This gave a gummy precipitate which was extracted into ethyl acetate (x4) and the combined organic extract was then washed (brine), dried (Na₂SO₄) and evaporated to give a gum. Flash chromatography (Si0₂/acetonitrilemethanol; 95:5 then 80:20) of this material gave the pure product (968 mg, 66%) as a white foam.

The foam was taken up in CH₂CI₂ and treated with excess ethanolic HCI. The solution was evaporated and the residue was again taken up in excess ethanolic HCI. Evaporation of the solution gave a light brown foam which was crystallized from hot methanol-acetone to give the hydrochloride (916 mg) as a white powder: m.p. 169-172 °C (dec); IR (KBr) 3400, 1632, 1550, 1275 cm⁻¹; ¹H nmr (200 MHz, CDCIa) δ 11.68 (br s, 1 H), 10.89 (s, 1 H), 8.66 (s, 1 H), 8.20 (s, 1 H), 7.56 (d, J = 7.4 Hz, 1 H), 7.33 (d, J = 7.7 Hz, 1 H), 7.21 (d, J = 2.3 Hz), 7.10-6.93 (m, 2H), 5.03-4.96 (m, 2H), 3.91 (s, 3H), 3.85-3.75 (m, 2H), 3.54-3.25 (m, 5H), 2.88-2.60 (m, 2H), 2.51-2.35 (m, 1 H), 1.88-1.81 (m, 1 H), 1.38 (d, J = 6.5 Hz, 3H).

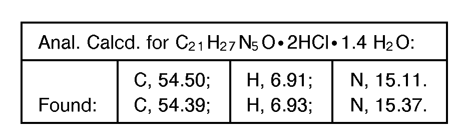

### Example 21

### 1-[3-(1 H-indol-3-yl)propyl]-4-(5-methoxy-4-pyrimidinyl)-3-methyl-piperazine

A mixture of 3-(1H-indol-3-yl)propyl) 4-methylbenzenesulfonate (1.30 g, 4.0 mmol), 1-(5-methoxy-4-pyrimidinyl)-2-methylpiperazine (0.80 g, 3.9 mmol), freshly pulverized KI (0.66 g, 4.0 mmol) and diisopropylethylamine (3.5 mL, 20 mmol) in 25 mL of dry acetonitrile was heated to reflux under Ar for 18 h. The reaction mixture was then diluted with ethyl acetate, washed (H₂0, brine), dried (Na₂S0₄) and evaporated to give a gum. The gum was chromatographed (Si0₂/ethyl acetate, then 5% MeOH-ethyl acetate) to give the title compound (0.75 g, 54%) as a foam. The foam was taken up in excess methanolic

### ² J. Szmuskovicz, etal., J. Am. Chem. Soc. 79, 2819 (1957).

HCI and the solution was evaporated and the residue was crystallized from methanol-isopropanol-ether to give 0.50 g of a solid. This was recrystallized from methanol-ether to give the hydrochloride (0.30 g) as an off-white solid: m.p. 185 ° C (dec); IR (KBr) 3400, 1630, 1544, 1500 cm⁻¹; ¹H nmr (200 MHz, d₆-DMSO) δ 11.00 (br s, 1 H), 10.88 (s, 1 H), 8.63 (s, 1 H), 8.20 (s, 1 H), 7.53 (d, J = 7.6 Hz, 1 H), 7.33 (d, J = 7.7 Hz, 1 H), 7.19 (d, J = 2.1 Hz, 1 H), 7.10-6.93 (m, 2H), 5.20 (br s, 1 H), 4.90-4.82 (m, 1 H), 3.90 (s, 3H), 3.70-3.48 (m, 3H), 3.11 (br s^{g} 4H), 2.76-2.69 (m, 2H), 2.16-2.13 (m, 2H), 1.51 (d, J = 7.1 Hz, 3H).

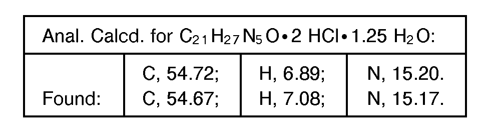

### Example 22

### 1-[3-(5-Methanesulfonamido-1 H-indol-3yl)propyl]-4-(5-methoxy-4-pyrimidinyl)piperazine

To a solution of 0.127 g (0.343 mmol, 1.0 equiv.) of mesylate (Example 13) in 3 mL of acetonitrile was added 0.12 mL (0.686 mmol, 2.0 equiv.) of N,N-diisopropylethylamine, 0.006 g (0.034 mmol, 0.1 equiv.) of potassium iodide and 0.133 g (0.686 mmol, 2.0 equiv.) of 1-(5-methoxy-4-pyrimidinyl)piperazine. The reaction mixture was heated to reflux for 16 hours. The reaction was cooled to room temperature and poured into saturated aqueous sodium bicarbonate. The aqueous layer was extracted with two five mL portions of ethyl acetate. The combined organic layers were dried over anhydrous sodium sulfate, filtered and concentrated in vacuo to yield 0.250 g of a yellow oil. This was chromatographed using 10% methanol in methylene chloride containing 1% concentrated aqueous ammonia to yield 0.0795 g (52%) of the base as an oil.

To a solution of 0.211 g (0.475 mmol, 1.0 equiv.) of the base in 5 mL of ethanol was added 0.95 mL (0.95 mmol, 2.0 equiv.) of 1 M HCI. The solution was concentrated to afford tan crystals. Recrystallization from hot ethanol yielded 0.1385 g of the hydrochloride ethanolate, C₂₁H₂₈N₆O₃S·2.5HCI·1.4EtOH, m.p. 150-152 _{°} C.

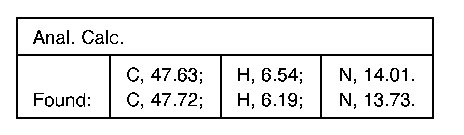

By the appropriate modification of these experimental procedures, additional products of Formula I can be obtained by one skilled in organic chemical synthesis.

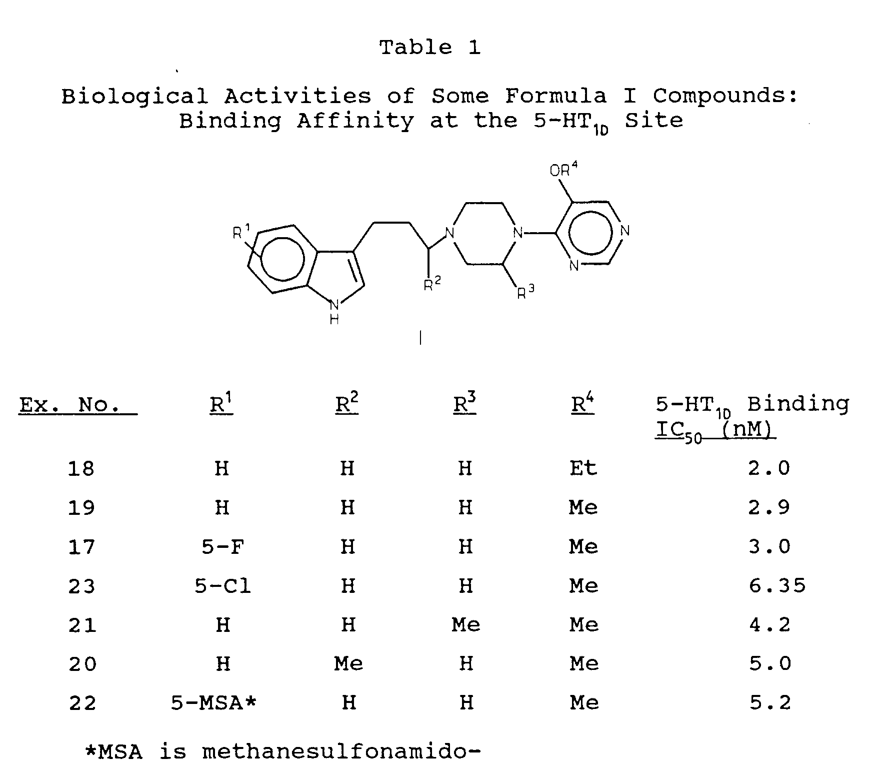

Some additional compounds similar to those defined hereinbefore by Formula I also have antimigraine properties. For instance, these compounds also display selective binding and agonist properties at the 5-HT_{1D} sub-type of serotonin receptor. In general the entire series displays potent interaction at that receptor with nearly all compounds having IC₅₀ values lower than 20 nmolar and the more potent members of the series having IC₅₀ values less than 5 nmolar. The significance of these IC₅₀ test values for 5-HT_{1D} binding sites, as it relates to antimigraine potential, has already been discussed for Formula I compounds.

Formula XI (shown below) expands the scope of the invention to include these additional compounds which have the structure given here as Formula I'. The additional subject matter comprises compounds of Formula I' 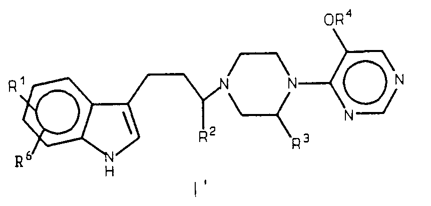 or a pharmaceutically acceptable acid addition salt and/or solvate thereof wherein
R¹ is lower alkoxy, phenyl-lower alkoxy, hydroxy, and 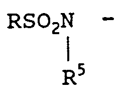
in which R is lower alkyl and R⁵ is hydrogen or lower alkyl;
R² and R³ are independently selected from hydrogen and lower alkyl;
R⁴ is lower alkyl; and
R⁶ is hydrogen and halogen.
Thus the present redefined invention comprehends a compound of Formula XI 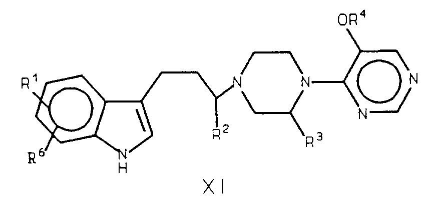
or a pharmaceutically acceptable acid addition salt and/or solvate thereof wherein
R¹ is selected from hydrogen, halogen, lower alkoxy, phenyl-lower alkoxy, hydroxy, and 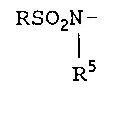 in which R is lower alkyl and R⁵ is hydrogen and lower alkyl;
R² and R³ are independently selected from hydrogen and lower alkyl;
R⁴ is lower alkyl; and
R⁶ is hydrogen and halogen.

As can be seen, Formula XI encompasses both Formula I and Formula I' compounds. The preferred solvate form of compounds of Formula XI are hydrates, i.e. compounds complexed with water of hydration.

The compounds of Formula I' can be obtained by appropriate modification of synthetic procedures outlined in Scheme 1 and exemplified specifically as experimental examples hereinabove. In addition, synthetic procedures described hereinbelow are also useful for preparation of Formula I' and, with obvious modifications, Formula I compounds.

Scheme 2 depicts some additional synthetic schemes which represent variation of Process #1 of Scheme 1 which has been previously outlined and described. The intermediate compounds of Formula II or V are then carried on synthetically according to Scheme 1.

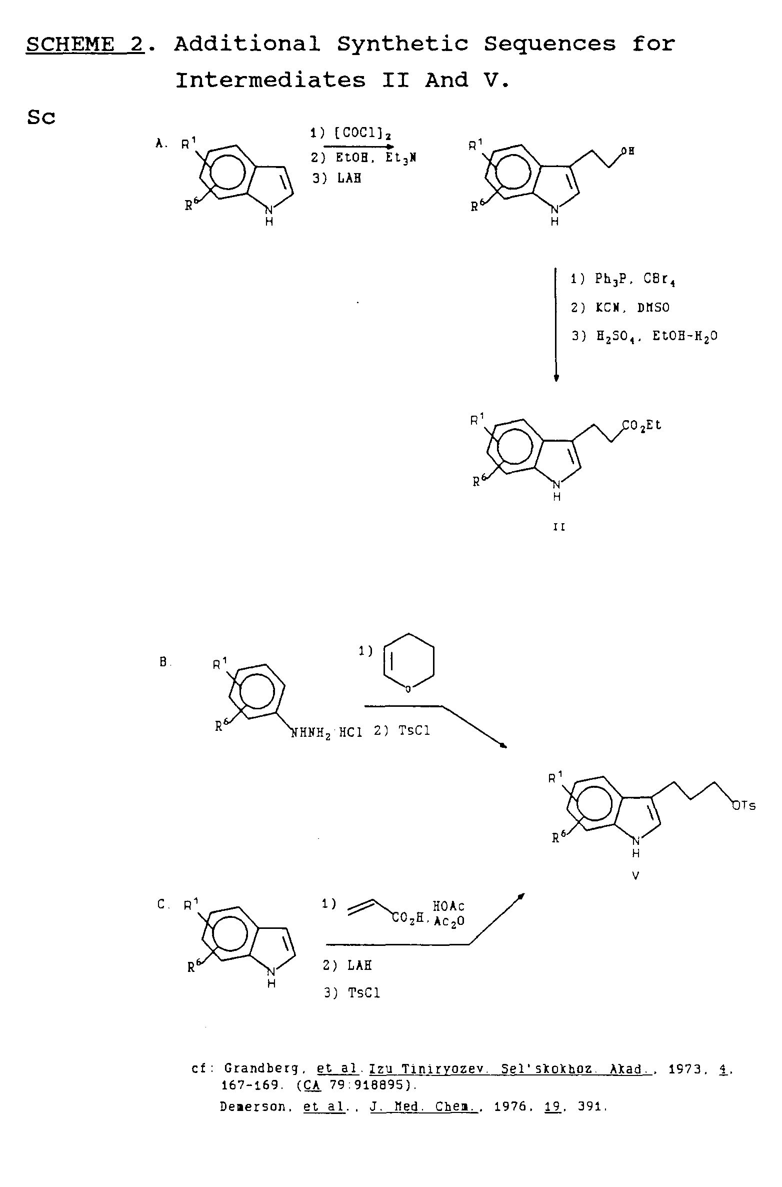

Scheme 3 demonstrates a synthesis for Formula XI products wherein R² is lower alkyl.

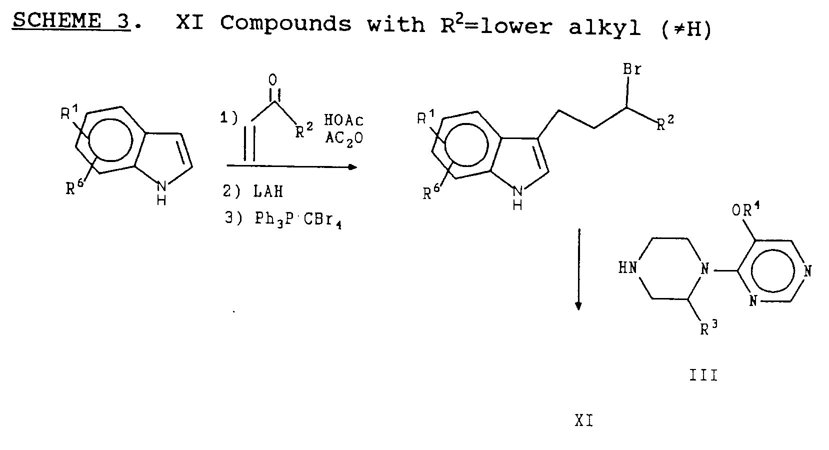

In Schemes 2 and 3, R^{I-}R⁶ are as defined for compounds of Formula XI. The Grandberg, et al., and Demerson, et al. references describe indole preparations employing phenylhydrazines and dihydropyrans and dihydrofurans. In Scheme 3, a selected indole is alkylated with an R²⁻vinylketone which is reduced to the corresponding alcohol and then converted to the bromo derivative by treatment with the triphenylphosphine-carbon tetrabromide complex. Reaction of this bromo intermediate with compound III gives a desired XI product having a lower alkyl R² group.

There is then the following unitary process for production of Formula XI compounds. This process comprises reacting a compound of Formula XV 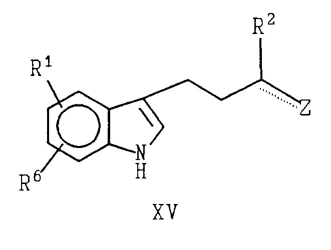 wherein R¹ is selected from hydrogen, halogen, lower alkoxy, phenyl-lower alkoxy, hydroxy, and 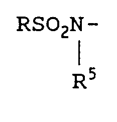 with R being lower alkyl and R⁵ being hydrogen and lower alkyl;
R² is hydrogen and lower alkyl;
R⁶ is hydrogen and halogen;
Z is selected from oxygen or an organic leaving group fragment such as tosyl, mesyl, halide, sulfate, phosphate, and
   the dotted line is either a covalent bond, as when Z is oxygen and a double bond between carbon and oxygen is required, or no bond, as when Z is a leaving group fragment requiring only a single bond and a hydrogen atom is bonded to the terminal carbon atom; with a compound of Formula III: 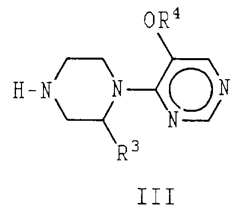 wherein R³ is hydrogen and lower alkyl; and
R⁴ is lower alkyl
   provided that if Z is oxygen, the reaction is carried out in the presence of a reducing agent.

Additional experimental procedures follow and they provide further guidance for the preparation compounds of the present invention.

### Example 23

### A. 5-(Methylsulfonyl)methylaminoindole

To a solution of 15.22 g (72.4 mmol, 1.0 equiv.) of 5-(methylsulfonyl)aminoindole in 400 mL of anhydrous THF at 0 ° C was added 32 mL (79.6 mmol, 1.1 equiv.) of a 2.5 M solution of n-BuLi in hexane dropwise. Stirring was continued for 30 minutes at 0 ° C followed by dropwise addition of 11.3 g (79.6 mmol, 1.1 equiv.) of methyl iodide. The reaction mixture was allowed to warm to room temperature and stir for 66 h. The mixture was poured into 1 L of ethyl acetate, washed with five 200 mL portions of 1 N NaOH and 200 mL of saturated aqueous sodium chloride. The organic layer was dried over anhydrous potassium carbonate, filtered and concentrated in vacuo to obtain 16.22 g (100%) of 5-(methylsulfonyl) methylaminoin- dole.

### B. 1-[3-[5-(Methylsulfonyl)amino-1-H-indol-3-yl]propyl] -4-(5-methoxy-4-pyrimidinyl)-3-methylpiperazine.

To a solution of 1.68 g (4.85 mmol, 1.0 equiv.) of 3-[5-(methylsulfonyl)aminoindolyl]propanol methanesulfonate in 20 mL of acetonitrile was added 2.50 g (19.4 mmol, 4.0 equiv.) of N,N-diisopropylethylamine, 0.01 g (0.0485 mmol, 0.01 equiv.) of potassium iodide and 2.02 g (9.7 mmol, 2.0 equiv.) of 4-(5-methoxy-4-pyrimidinyl)-3-methylpiperazine. The reaction was heated to reflux for 48 h. The solution was cooled and poured into a mixture of 50 mL of ethyl acetate and 50 mL of saturated sodium bicarbonate. The organic layer was separated and the aqueous layer reextracted with three 25 mL portions of ethyl acetate. The combined organic layers were washed with 25 mL of saturated aqueous sodium chloride, dried over anhydrous potassium carbonate, filtered and concentrated in vacuo. The residue was chromatographed with 96:4 methylene chloride: methanol to yield 0.82 g (37%) of the desired material. The hydrochloride was prepared by addition of 2 equivalents of 9.68 M HCI in ethanol to the base in ethanol. The solution was concentrated and the hydrochloride recrystallized from ethanol. The salt was dried under high vacuum for four h at 65 _{°} C to yield 1-[3-[5-(methylsulfonyl)amino-1-H-indol-3yl]propyl]-4-(5-methoxy-4-pyrimidinyl)-3-methylpiperazine hydrochloride hydrate, m.p. 205-206 °C.

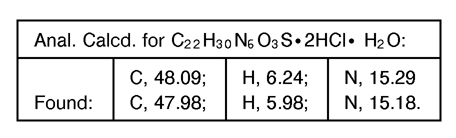

### Example 24

### 1-[3-(5-Hydroxy-1 H-indol-3-yl)propyl]-4-(5-methoxy-4-pyrimidinyl)piperazine

A mixture of 1-[3-(5-benzyloxy-1H-indol-3-yl)propyl]-4-(5-methoxy-4-pyrimidinyl)piperazine (Ex. 33; 1.40 g, 3.06 mmol) and 10% Pd(OH)₂/C (0.85 g) in 25 mL of ethanol was hydrogenated at 30-40 psi in a Parr shaker for 3.5 h. The mixture was filtered and then fresh catalyst (0.75 g) was added to the filtrate and hydrogenation was resumed at ca 45 psi for 22 h. The resulting mixture was filtered through Celite and the filtrate was evaporated to give a foam (0.995 g, 88%). The foam was taken up in methanolic HCI, whereupon a white solid precipitated. The solid was filtered, washed with cold methanol and then ether, and dried in vacuo to give the hydrochloride as a slightly pinkish solid (0.59 g), mp 215 ° C; IR (KBr) 3320 (Br) 1630, 1550 cm-¹; 7.07 (s, 1 H), 6.80 (d, J = 2.1 Hz, 1 H), 6.59 (dd, J = 2.2, 8.6 Hz, 1 H), 4.91-4.84 (m, 2H), 3.90 (s, 3H), 3.8-3.4 (m, 7H), 3.2-3.0 (m, 4H), 2.69-2.62 (m, 2H), 2.2-2.0 (m, 2H).

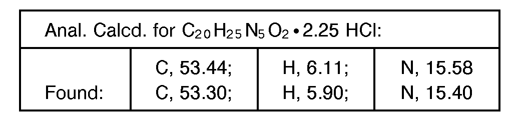

### Example 25

### 3-[3-[4-(5-Methoxy-4-pyrimidinyl)-1-piperazinyl]pentyl] indole

A mixture of 3-(3-bromopentyl)indole (1.33 g, 5.0 mmol 1-(5-methoxy-4-pyrimidyl)piperazine (2.00 g, 10 mmol) finely pulverized K₂C0₃ (0.70 g, 5.1 mmol) and finely pulverized KI (0.90 g, 5.1 mmol) in 20 mL of acetonitrile was heated to reflux under Ar for 5 h. The cooled reaction mixture was diluted with EtOAc, washed (H₂0, brine), dried (Na₂S0₄) and evaporated to give a gum. Chromatography of this material (Si0₂/EtOAc then 10% MeOH-EtOAc) affords the product (0.65 g, 34%) as a gum. This gum was taken up in ether and then methanotic HCI was added. The resulting solid was filtered, washed with Et₂0 and dried in vacuo to give an off-white solid (0.50 g), mp 145 ° C (dec); IR (KBr) 3400, 1633, 1550 cm-1;

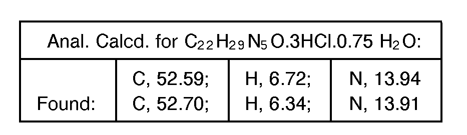

By the appropriate modification of the foregoing schemes and experimental procedures, additional products of Formula XI can be obtained by one skilled in organic chemical synthesis.

### Example 26

1-[3-[5-(Methylsulfonyl)methylamino-1-H-indol-3-yl] propyl]-4-(5-methoxy-4-pyrimidinyl)piperazine hydrochloride hydrate, m.p. 227-230 ° C was prepared in a similar manner starting from 5-(methylsulfonyl)-methylaminoindole.

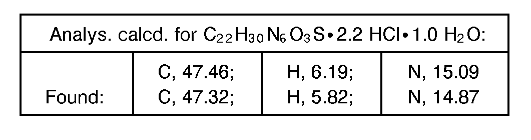

### Example 27

1-[3-[5-(Methylsulfonyl)methylamino-1-H-indol-3-yl] propyl]-4-(5-methoxy-4-pyrimidinyl)-3-methylpiperazine hydrochloride hydrate, m.p. 215-218 ° C was prepared in a similar manner starting from 5-(methylsulfonyl)-methylaminoindole.

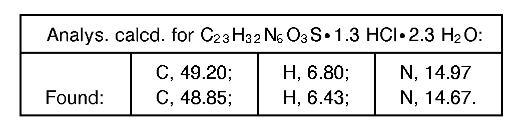

### Example 28

1-[3-[5-(Ethylsulfonyl)amino-1-H-indol-3-yl]propyl]-4-(5-methoxy-4-pyrimidinyl)piperazine hydrochloride hydrate, m.p. 140-145 ° C was prepared in a similar manner starting from 5-(ethylsulfony)aminoindole.

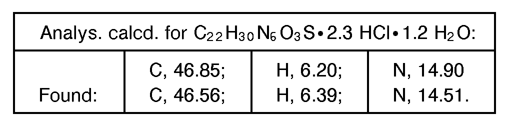

### Example 29

1-[3-[5-(Ethylsulfonyl)methylamino-1-H-indol-3-yl]propyl]-4-(5-methoxy-4-pyrimidinyl)piperazine hydrochloride hydrate, m.p. 223-225 ° C was prepared in a similar manner starting from 5-(ethylsulfony)-methylaminoindole.

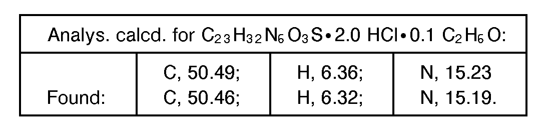

### Example 30

1-[3-[5-(Ethylsulfonyl)methylamino-1H-indol-3-yl]propyl]-4-(5-methoxy-4-pyrimidinyl)-3-methylpiperazine hydrochloride hydrate, m.p. 205-207 ° C was prepared in a similar manner starting from 5-(ethylsulfonyl)-methylaminoindole.

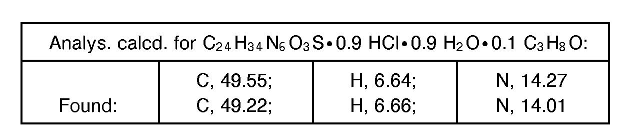

### Example 31

1-[3-[5-(Ethylsulfonyl)amino-1H-indol-3-yl]propyl]-4-(5-methoxy-4-pyrimidinyl)-3-methylpiperazine hydrochloride hydrate, m.p. 212-214 ° C was prepared in a similar manner starting from 5-(ethylsulfonyl)aminoindole.

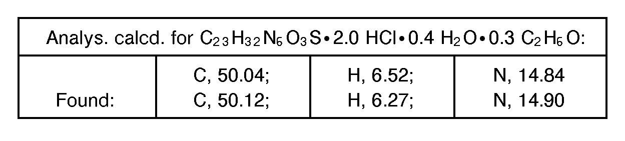

Additional Compound XI products and their 5-HT_{1D} binding activities are displayed in Tables 2 and 3.

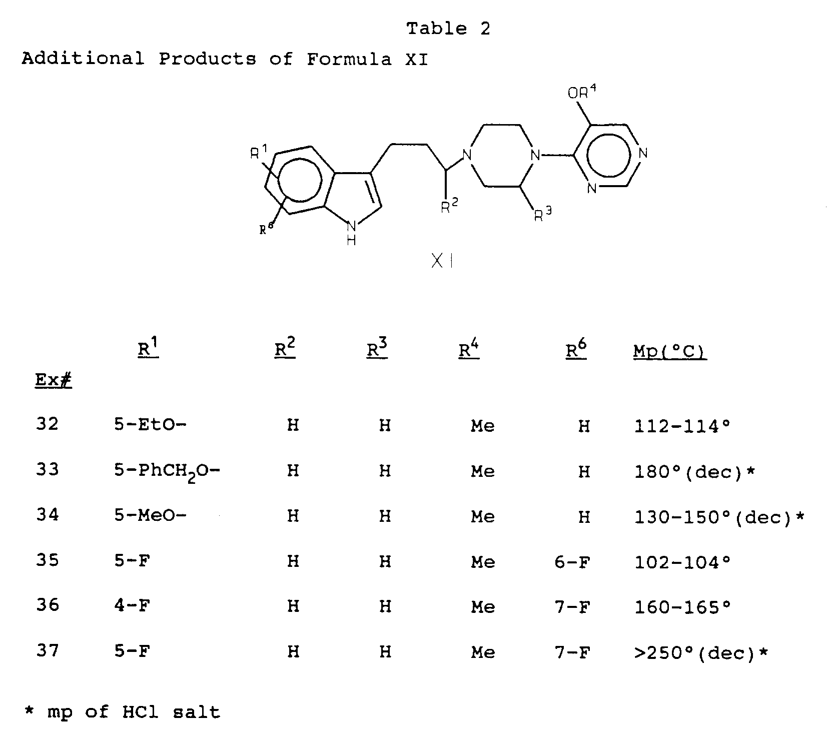

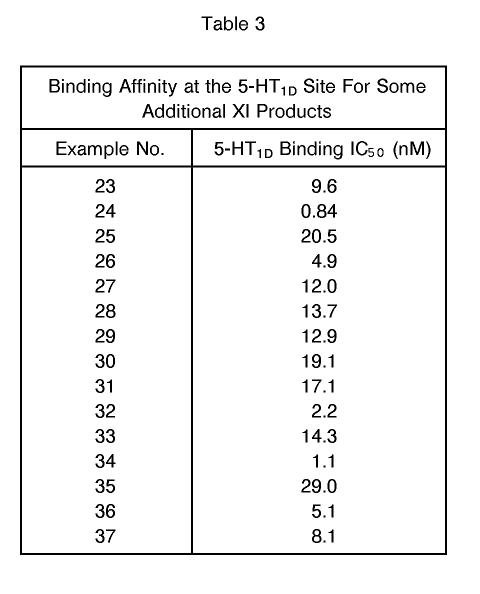

## Claims (Claims for the following Contracting State(s) : AT, BE, CH, DE, DK, FR, GB, IT, LI, LU, NL, SE)

1. A compound of the following formula: 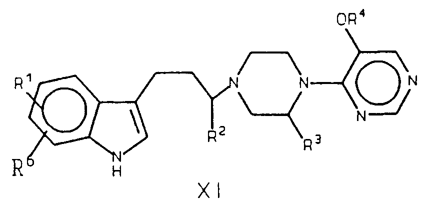 wherein
R¹ is selected from hydrogen, halogen, C₁-C₄ alkoxy, phenyl-C₁-C₄ alkoxy, hydroxy, and 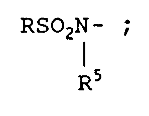
R², R³ and R⁵ are independently selected from hydrogen and C₁-C₄ alkyl;
R⁴ is C₁ -C₄ alkyl;
R⁶ is hydrogen and halogen.
or a pharmaceutically
acceptable acid addition salt and/or solvate and/or stereoisomer thereof.

2. A compound of Formula I or a pharmaceutically acceptable acid addition salt thereof 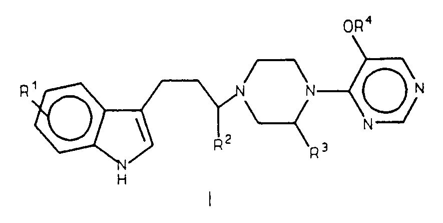 wherein
R¹ is selected from hydrogen, halogen; or 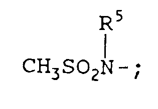
R², R³ and R⁵ are independently selected from hydrogen and C₁-C₄ alkyl; and
R⁴ is C₁ -C₄ alkyl.

3. A compound according to claim 1 or 2 wherein R¹ is hydroxyl.

4. A compound according to claim 1 selected from 4-(5-ethoxy-4-pyrimidinyl)-1-[3-(1H-indol-3-yl)propyl]-piperazine; 1-[3-(lH-indol-3-yl)propyl]-4-(5-methoxy-4-pyrimidinyl)-piperazine; 1-[3-(5-fluoro-lH-indol-3-yl)propyl]-4-(5-methoxy-4-pyrimidinyl)-piperazine; 1-[3-(5-chloro-1H-indol-3-yl)propyl]-4-(5-methoxy-4-pyrimidinyl)-piperazine; 1-[3-(1H-indol-3-yl)propyl]-4-(5-methoxy-4-pyrimidinyl)-3-methylpiperazine;1-[3-(1H-indol-3-yl)butyl]-4-(5-methoxy-4-pyrimidinyl)-piperazine; 1-[3-[5-(methylsulfonyl)-amino-1H-indol-3-yl]propyl]-4-(5-methoxy-4-pyrimidinyl) piperazine; 1-[3-[5-(methylsulfonyl)-amino-1H-indol-3-yl]propyl]-4-(5-methoxy-4-pyrimidinyl)-3-methylpiperazine; 1-[3-(5-hydroxy-1H-indol-3-yl)propyl]-4-(5-methoxy-4-pyrimidinyl)-piperazine; 3-[3-[4-(5-methoxy-4-pyrimidinyl)-1-piperazinyl]pentyl]indole; 1-[3-[5-methylsulfonyl)-methylamino-1H-indol-3-yl]propyl]-4-(5-methoxy-4-pyrimidinyl)piperazine; 1-[3-[5-methylsulfonyl)-methylamino-1H-indol-3-yl]propyl]-4-(5-methoxy-4-pyrimidinyl)-3-methylpiperazine; 1-[3-[5-ethylsulfonyl)amino-1H-indol-3-yl]propyl]-4-(5-methoxy-4-pyrimidinyl)piperazine; 1-[3-[5-et hylsulfonyl) methylamino-1H-indol-3-yl]propyl]-4-(5-methoxy-4-pyrimidinyl)piperazine; 1, 1-[3-[5-ethylsulfonyl)-methylamino-1H-indol-3-yl]propyl]-4-(5-methoxy-4-pyrimidinyl)-3-methylpiperazine; 1-[3-[5-ethylsulfonyl)amino-1H-indol-3-yl]propyl]-4-(5-methoxy-4-pyrimidinyl)-3-methylpiperazine; 1-[3-(5-ethoxy)-1H-in- dol-3-yl] propyl]-4-(5-methoxy-4-pyrimidinyl)piperazine; 1-[3-(5-benzyloxy-1H-indol-3-yl)propyl]-4-(5-methoxy-4-pyrimidinyl) piperazine; 1-[3-(5-methoxy-1H-indol-3-yl)propyl]-4-(5-methoxy-4-pyrimidinyl)-piperazine; 1-[3-(5,6-difluoro-1H-indol-3-yl)propyl]-4-(5-methoxy-4-pyrimidinyl)piperazine; 1-[3-(4,7-difluoro-1 H-indol-3-yl)propyl]-4-(5-methoxy-4-pyrimidinyl)piperazine; 1-[3-(5,7-difluoro-1H-indol-3-yl)-propyl]-4-(5-methoxy-4-pyrimidinyl)piperazine, or a pharmaceutically acceptable acid addition salt thereof, and/or solvate and/or stereoisomer thereof.

5. 1-[3-(5-fluoro-1H-indol-3-yl)propyl]-4-(5-methoxy-4-pyrimidinyl)piperazine or a pharmaceutically acceptable acid addition salt and/or solvate thereof.

6. A pharmaceutical formulation which comprises as an active ingredient a compound, as claimed in any one of claims 1-5, associated with one or more pharmaceutically acceptable carriers, excipients or diluents therefore.

7. A pharmaceutical formulation according to claim 6 wherein the formulation is in unit dosage form.

8. A pharmaceutically formulation which comprises as an active ingredient 1-[3-(5-fluoro-1H-indol-3-yl)-propyl]-4-(5-methoxy-4-pyrimidinyl)piperazine associated with one or more pharmaceutically acceptable carriers, excipients or diluents thereof.

9. A pharmaceutical formulation according to claim 8 wherein the formulation is in unit dosage form.

10. A compound as claimed in any of one of claims 1-5 for use as an antimigraine agent.

11. 1-[3-(5-fluoro-lH-indol-3-yl)propyl]-4-(5-methoxy-4-pyrimidinyl)piperazine for use as an antimigraine agent.

12. A pharmaceutical formulation as claimed in any of claims 6-9 for use as an antimigraine formulation.

13. A process for preparing a compound as claimed in any one of claims 1-5 which comprises reacting a compound of Formula XV 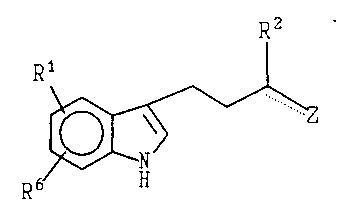 wherein R¹, R² and R⁶ are as defined in claim 1;
Z is selected from oxygen or a leaving group fragment selected from tosyl, mesyl, halide, sulfate and phosphate; and
the dotted line is either a covalent bond as when Z is oxygen or no bond as when Z is a leaving group fragment and the carbon atom is then bonded to another hydrogen;
with a compound of formula III 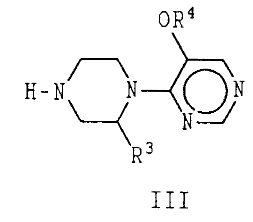
wherein R³ and R⁴ are as defined in claim 1,
provided that if Z is oxygen, the reaction is carried out in the presence of a reducing agent.

## Claims (Claims for the following Contracting State(s) : ES)

1. A process for preparing a compound of the following formula: 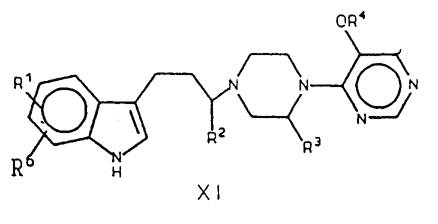 wherein
R¹ is selected from hydrogen, halogen, C₁-C₄ alkoxy, phenyl-C₁-C₄ alkoxy, hydroxy, and 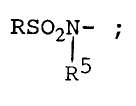
R², R³ and R⁵ are independently selected from hydrogen and C₁-C₄ alkyl;
R⁴ is C₁ -C₄ alkyl;
R⁶ is hydrogen and halogen
or a pharmaceutically acceptable acid addition salt and/or solvate and/or stereoisomer thereof, which comprises reacting a compound of Formula XV 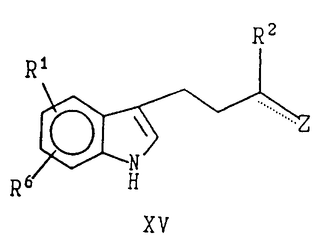 wherein R',R² and R⁶ are as defined above;
Z is selected from oxygen or a leaving group fragment selected from tosyl, mesyl, halide, sulfate and phosphate; and
the dotted line is either a covalent bond as when Z is oxygen or no bond as when Z is a leaving group fragment and the carbon atom is then bonded to another hydrogen; with a compound of formula III 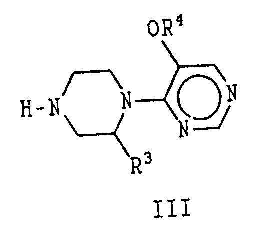 wherein R³ and R⁴ are as defined above,
provided that if Z is oxygen, the reaction is carried out in the presence of a reducing agent.

2. A process according to claim 1 for preparing a compound of Formula I or a pharmaceutically acceptable acid addition salt thereof 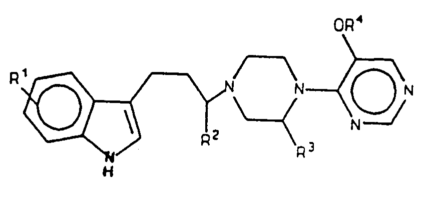 wherein
R¹ is selected from hydrogen, halogen; or 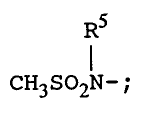
R², R³ and R⁵ are independently selected from hydrogen and C₁-C₄ alkyl; and
R⁴ is C₁ -C₄ alkyl.

3. A process according to claim 1 for preparing a compound as defined in claim 1 or 2 wherein R¹ is hydroxyl.

4. A process according to claim 1 for preparing a compound as defined in claim 1, wherein said compound is selected from 4-(5-ethoxy-4-pyrimidinyl)-1-[3-(1H-indol-3-yl)propyl]piperazine; 1-[3-(lH- indol-3-yl)propyl]-4-(5-methoxy-4-pyrimidinyl)-piperazine; 1-[3-(5-fluoro-1H-indol-3-yl)propyl]-4-(5-methoxy-4-pyrimidinyl)-piperazine;1-[3-(5-chloro-1H-indol-3-yl)propyl]-4-(5-methoxy-4-pyrimidinyl)-piperazine; 1-[3-(1H-indol-3-yl)propyl]-4-(5-methoxy-4-pyrimidinyl)-3-methylpiperazine; 1-[3-(1H-indol-3-yl)-butyl]-4-(5-methoxy-4-pyrimidinyl)-piperazine; 1-[3-[5-(methylsulfonyl)-amino-1H-indol-3-yl]propyl]-4-(5-methoxy-4-pyrimidinyl) piperazine; 1-[3-[5-(methylsulfonyl)-amino-1H-indol-3-yl]propyl]-4-(5-methoxy-4-pyrimidinyl)-3-methylpiperazine; 1-[3-(5-hydroxy-1 H-indol-3-yl)propyl]-4-(5-methoxy-4-pyrimidinyl)-piperazine; 3-[3-[4-(5-methoxy-4-pyrimidinyl)-1-piperazinyl]pentyl]indole; 1-[3-[5-methylsulfonyl)-methylamino-1H-indol-3-yl]propyl]-4-(5-methoxy-4-pyrimidinyl)piperazine; 1-[3-[5-methylsulfonyl)-methylamino-1 H-indol-3-yl]propyl]-4-(5-methoxy-4-pyrimidinyl)-3-methylpiperazine; 1-[3-[5-ethylsulfonyl)amino-1H-i ndol-3-yl]propyl]-4-(5-methoxy-4-pyrimidinyl)piperazine; 1-[3-[5-ethylsulfonyl) methylamino-1H-indol-3-yl]propyl]-4-(5-methoxy-4-pyrimidinyl)piperazine; 1, 1-[3-[5-ethylsulfonyl)-methylamino-1H-indol-3-yl]propyl]-4-(5-methoxy-4-pyrimidinyl)-3-methylpiperazine; 1-[3-[5-ethylsulfonyl)amino-1H-indol-3-yl]propyl]-4-(5-methoxy-4-pyrimidinyl)-3-methylpiperazine; 1-[3-(5-ethoxy)-1H-in- dol-3-yl]propyl]-4-(5-methoxy-4-pyrimidinyl)piperazine; 1-[3-(5-benzyloxy-1H-indol-3-yl)propyl]-4-(5-methoxy-4-pyrimidinyl) piperazine; 1-[3-(5-methoxy-1H-indol-3-yl)propyl]-4-(5-methoxy-4-pyrimidinyl)-piperazine; 1-[3-(5,6-difluoro-1H-indol-3-yl)propyl]-4-(5-methoxy-4-pyrimidinyl)piperazine; 1-[3-(4,7-difluoro-1 H-indol-3-yl)propyl]-4-(5-methoxy-4-pyrimidinyl)piperazine; 1-[3-(5,7-difluoro-1H-indol-3-yl)-propyl]-4-(5-methoxy-4-pyrimidinyl)piperazine, or a pharmaceutically acceptable acid addition salt thereof, and/or solvate and/or stereoisomer thereof.

5. A process for preparing a pharmaceutical formulation which comprises admixing a compound as defined in any one of claims 1-5 with one or more pharmaceutically acceptable carriers, excipients or diluents therefore.

6. A process according to claim 6 wherein the formulation is in unit dosage form.

7. A process for preparing a pharmaceutically formulation which comprises admixing 1-[3-(5-fluoro-lH- indol-3-yl)propyl]-4-(5-methoxy-4-pyrimidinyl)piperazine with one or more pharmaceutically acceptable carriers, excipients or diluents thereof.

8. A process according to claim 8 wherein the formulation is in unit dosage form.

9. A process according to any one of claims 6-9, wherein said pharmaceutical formulation is for use as an antimigraine formulation.

## Claims (Claims for the following Contracting State(s) : GR)

1. A compound of the following formula: 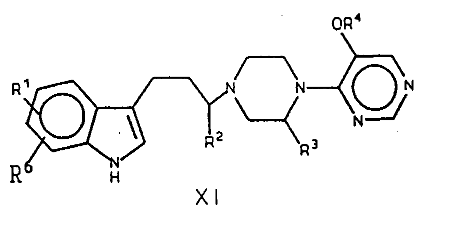 wherein
R¹ is selected from hydrogen, halogen, C₁-C₄ alkoxy, phenyl-C₁-C₄ alkoxy, hydroxy, and 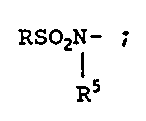
R², R³ and R⁵ are independently selected from hydrogen and C₁-C₄ alkyl;
R⁴ is C₁ -C₄ alkyl;
R⁶ is hydrogen and halogen.
or a pharmaceutically
acceptable acid addition salt and/or solvate and/or stereoisomer thereof.

2. A compound of Formula I or a pharmaceutically acceptable acid addition salt thereof 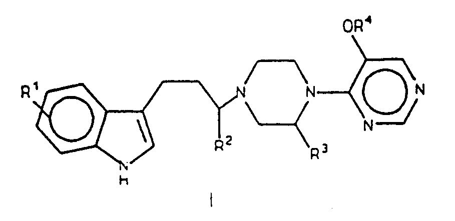 wherein
R¹ is selected from hydrogen, halogen; or 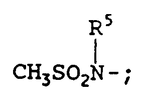
R², R³ and R⁵ are independently selected from hydrogen and C₁-C₄ alkyl; and
R⁴ is C₁ -C₄ alkyl.

3. A compound according to claim 1 or 2 wherein R¹ is hydroxyl.

4. A compound according to claim 1 selected from 4-(5-ethoxy-4-pyrimidinyl)-1-[3-(1H-indol-3-yl)propyl]-piperazine; 1-[3-(lH-indol-3-yl)propyl]-4-(5-methoxy-4-pyrimidinyl)-piperazine; 1-[3-(5-fluoro-lH-indol-3-yl)propyl]-4-(5-methoxy-4-pyrimidinyl)-piperazine; 1-[3-(5-chloro-1H-indol-3-yl)propyl]-4-(5-methoxy-4-pyrimidinyl)-piperazine; 1-[3-(1H-indol-3-yl)propyl]-4-(5-methoxy-4-pyrimidinyl)-3-methylpiperazine;1-[3-(1H-indol-3-yl)butyl]-4-(5-methoxy-4-pyrimidinyl)-piperazine; 1-[3-[5-(methylsulfonyl)-amino-1H-indol-3-yl]propyl]-4-(5-methoxy-4-pyrimidinyl) piperazine; 1-[3-[5-(methylsulfonyl)-amino-1H-indol-3-yl]propyl]-4-(5-methoxy-4-pyrimidinyl)-3-methylpiperazine; 1-[3-(5-hydroxy-1H-indol-3-yl)propyl]-4-(5-methoxy-4-pyrimidinyl)-piperazine; 3-[3-[4-(5-methoxy-4-pyrimidinyl)-1-piperazinyl]pentyl]indole; 1-[3-[5-methylsulfonyl)-methylamino-1H-indol-3-yl]propyl]-4-(5-methoxy-4-pyrimidinyl)piperazine; 1-[3-[5-methylsulfonyl)-methylamino-1H-indol-3-yl]propyl]-4-(5-methoxy-4-pyrimidinyl)-3-methylpiperazine; 1-[3-[5-ethylsulfonyl)amino-1H-indol-3-yl]propyl]-4-(5-methoxy-4-pyrimidinyl)piperazine; 1-[3-[5-et hylsulfonyl) methylamino-1H-indol-3-yl]propyl]-4-(5-methoxy-4-pyrimidinyl)piperazine; 1, 1-[3-[5-ethylsulfonyl)-methylamino-1H-indol-3-yl]propyl]-4-(5-methoxy-4-pyrimidinyl)-3-methylpiperazine; 1-[3-[5-ethylsulfonyl)amino-1H-indol-3-yl]propyl]-4-(5-methoxy-4-pyrimidinyl)-3-methylpiperazine; 1-[3-(5-ethoxy)-1H-in- dol-3-yl] propyl]-4-(5-methoxy-4-pyrimidinyl)piperazine; 1-[3-(5-benzyloxy-1H-indol-3-yl)propyl]-4-(5-methoxy-4-pyrimidinyl) piperazine; 1-[3-(5-methoxy-1H-indol-3-yl)propyl]-4-(5-methoxy-4-pyrimidinyl)-piperazine; 1-[3-(5,6-difluoro-1H-indol-3-yl)propyl]-4-(5-methoxy-4-pyrimidinyl)piperazine; 1-[3-(4,7-difluoro-1 H-indol-3-yl)propyl]-4-(5-methoxy-4-pyrimidinyl)piperazine; 1-[3-(5,7-difluoro-1H-indol-3-yl)-propyl]-4-(5-methoxy-4-pyrimidinyl)piperazine, or a pharmaceutically acceptable acid addition salt thereof, and/or solvate and/or stereoisomer thereof.

5. 1-[3-(5-fluoro-1H-indol-3-yl)propyl]-4-(5-methoxy-4-pyrimidinyl)piperazine or a pharmaceutically acceptable acid addition salt and/or solvate thereof.

6. A process for preparing a pharmaceutical formulation which comprises admixing a compound as defined in any one of claims 1-5 with one or more pharmaceutically acceptable carriers, excipients or diluents therefore.

7. A process according to claim 6 wherein the formulation is in unit dosage form.

8. A process for preparing a pharmaceutically formulation which comprises admixing 1-[3-(5-fluoro-lH- indol-3-yl)propyl]-4-(5-methoxy-4-pyrimidinyl)piperazine with one or more pharmaceutically acceptable carriers, excipients or diluents thereof.

9. A process according to claim 8 wherein the formulation is in unit dosage form.

10. A compound as claimed in any one of claims 1-5 for use as an antimigraine agent.

11. 1-[3-(5-fluoro-lH-indol-3-yl)propyl]-4-(5-methoxy-4-pyrimidinyl)piperazine for use as an antimigraine agent.

12. A process according to any one of claims 6-9, wherein said pharmaceutical formulation is for use as an antimigraine formulation.

13. A process for preparing a compound as claimed in any one of claims 1-5 which comprises reacting a compound of Formula XV 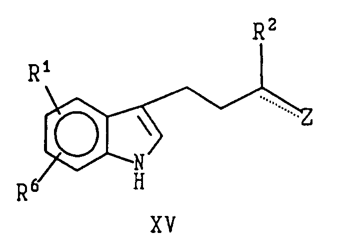 wherein R¹, R² and R⁶ are as defined in claim 1;
Z is selected from oxygen or a leaving group fragment selected from tosyl, mesyl, halide, sulfate and phosphate; and
the dotted line is either a covalent bond as when Z is oxygen or no bond as when Z is a leaving group fragment and the carbon atom is then bonded to another hydrogen;
with a compound of formula III 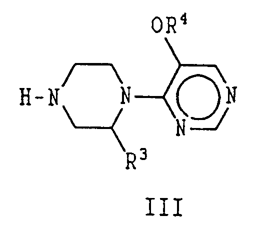
wherein R³ and R⁴ are as defined in claim 1,
provided that if Z is oxygen, the reaction is carried out in the presence of a reducing agent.

## Patentansprüche (Patentansprüche für folgende(n) Vertragsstaat(en) : AT, BE, CH, DE, DK, FR, GB, IT, LI, LU, NL, SE)

1. Verbindung der folgenden Formel: 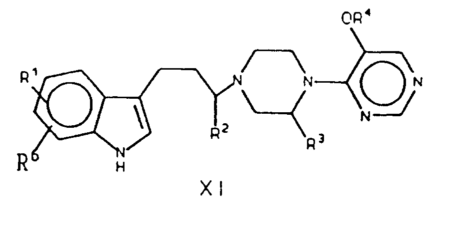 worin
R¹ ausgewählt ist unter Wasserstoff, Halogen, C₁-C₄-Alkoxy, Phenyl-C₁-C₄-alkoxy, Hydroxy und 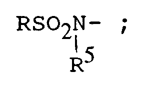
R², R³ und R⁵ unabhängig voneinander ausgewählt sind unter Wasserstoff und C₁-C₄-Alkyl;
_{R}⁴ für C₁-C₄-Alkyl steht;
R⁶ für Wasserstoff und Halogen steht,
oder ein pharmazeutisch akzeptables Säureadditionssalz und/oder Solvat und/oder Stereoisomer davon.

2. Verbindung der Formel 1 oder ein pharmazeutisch akzeptables Säureadditionssalz davon 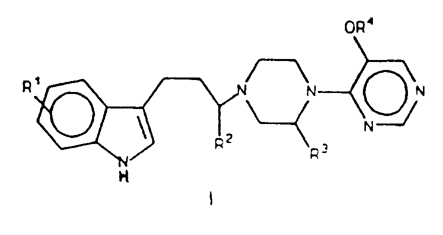 worin
R¹ ausgewählt ist unter Wasserstoff, Halogen oder 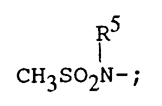
R², R³ und R⁵ unabhängig voneinander ausgewählt sind unter Wasserstoff und C₁-C₄-Alkyl; und
_{R}⁴ für C₁-C₄-Alkyl steht.

3. Verbindung nach Anspruch 1 oder 2, wobei R¹ für Hydroxy steht.

4. Verbindung nach Anspruch 1, ausgewählt unter 4-(5-Ethoxy-4-pyrimidinyl)-1-[3-(1H-indol-3-yl)propyl]-piperazin; 1-[3-(1H-Indol-3-yl)propyl]-4-(5-methoxy-4-pyrimidinyl)-piperazin; 1-[3-(5-Fluor-1H-indol-3-yl)-propyl]-4-(5-methoxy-4-pyrimidinyl)piperazin; 1-[3-(5-Chlor-1H-indol-3-yl)propyl]-4-(5-methoxy-4-pyrimidinyl)piperazin; 1-[3-(lH-Indol-3-yl)propyl]-4-(5-methoxy-4-pyrimidinyl)-3-methylpiperazin; 1-[3-(1H-Indol-3-yl)butyl]-4-(5-methoxy-4-pyrimidinyl)piperazin; 1-[3-[5-(methylsulfonyl)-amino-1H-indol-3-yl]-propyl]-4-(5-methoxy-4-pyrimidinyl)piperazin; 1-[3-[5-(Methylsulfonyl)-amino-1H-indol-3-yl]propyl]-4-(5-methoxy-4-pyrimidinyl)-3-methylpiperazin; 1-[3-(5-Hydroxy-1H-indo!-3-y!)propy!]-4-(5-methoxy-4-pyrimidinyl)-piperazin; 3-[3-[4-(5-Methoxy-4-pyrimidinyl)-1-piperazinyl]pentyl]indol; 1-[3-[5-Methylsulfonyl)-me- thylamino-1H-indol-3-yl]propyl]-4-(5-methoxy-4-pyrimidinyl)piperazin; 1-[3-[5-Methylsulfonyl)-methylami- no-1H-indol-3-yl]propyl]-4-(5-methoxy-4-pyrimidinyl)-3-methylpiperazin; 1-[3-[5-Ethylsulfonyl)-amino-1Hin dol-3-yl]propyl]-4-(5-methoxy-4-pyrimidinyl)piperazin; 1-[3-[5-Ethylsulfonyl)-methylamino-1H-indol-3-yl]propyl]-4-(5-methoxy-4-pyrimidinyl)piperazin; 1,1-[3-[5-Ethylsulfonyl)-methylamino-1H-indol-3-yl]-propyl]-4-(5-methoxy-4-pyrimidinyl)-3-met hylpiperazin; 1-[3-[5-Ethylsulfonyl)-amino-1H-indol-3-yl]-propyl]-4-(5-methoxy-4-pyrimidinyl)-3-methylpiperazin; 1-[3-(5-Ethoxy)-1H-indol-3-yl]propyl]-4-(5-me- thoxy-4-pyrimidinyl)piperazin; 1-[3-(5-Benzyloxy-1H-indol-3-yl)propyl]-4-(5-methoxy-4-pyrimidinyl)-piperazin; 1-[3-(5-Methoxy-1H-indol-3-yl)propyl]-4-(5-methoxy-4-pyrimidinyl)piperazin; 1-[3-(5,6-Difluor-1H-indol-3-yl)propyl]-4-(5-methoxy-4-pyrimidinyl)piperazin; 1-[3-(4,7-Difluor-1H-indol-3-yl)propyl]-4-(5-methoxy-4-pyrimidinyl)piperazin; 1-[3-(5,7-Difluor-1H-indol-3-yl)propyl]-4-(5-methoxy-4-pyrimidinyl)-piperazin, oder ein pharmazeutisch akzeptables Säureadditionssalz und/oder Solvat und/oder Stereoisomer davon.

5. 1-[3-(5-Fluor-1H-indol-3-yl)propyl]-4-(5-methoxy-4-pyrimidinyl)piperazin oder ein pharmazeutisch akzeptables Säureadditionssalz und/oder Solvat davon.

6. Pharmazeutische Formulierung, die als aktiven Bestandteil eine Verbindung nach einem der Ansprüche 1 bis 5, zusammen mit einem oder mehreren pharmazeutisch akzeptablen Trägern, Exzipienten oder Verdünnungsmitteln umfaßt.

7. Pharmazeutische Formulierung nach Anspruch 6, wobei die Formulierung in Dosiseinheitsform vorliegt.

8. Pharmazeutische Formulierung, die als aktiven Bestandteil 1-[3-(5-Fluor-1H-indol-3-yl)propyl]-4-(5-me- thoxy-4-pyrimidinyl)piperazin, zusammen mit einem oder mehreren pharmazeutisch akzeptablen Trägern, Exzipienten oder Verdünnungsmitteln umfaßt.

9. Pharmazeutische Formulierung nach Anspruch 8, wobei die Formulierung in Dosiseinheitsform vorliegt.

10. Verbindung nach einem der Ansprüche 1 bis 5 zur Verwendung als Antimigränemittel.

11. 1-[3-(5-Fluor-1H-indol-3-yl)propyl]-4-(5-methoxy-4-pyrimidinyl)piperazin zur Verwendung als Antimigränemittel.

12. Pharmazeutische Formulierung nach einem der Ansprüche 6 bis 9 zur Anwendung als Antimigräneformulierung.

13. Verfahren zur Herstellung einer Verbindung nach einem der Ansprüche 1 bis 5, wobei man eine Verbindung der Formel XV 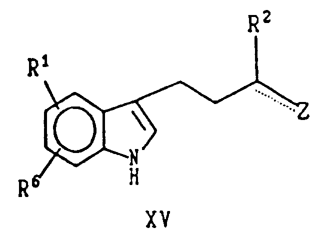 worin
R¹, R² und R⁶ wie in Anspruch 1 definiert sind;
Z ausgewählt ist unter Sauerstoff oder einer Abgangsgruppe, die ausgewählt ist unter Tosyl, Mesyl, Halogenid, Sulfat und Phosphat; und die gestrichelte Linie eine kovalente Bindung bedeutet, wenn Z für Sauerstoff steht, oder keine Bindung bedeutet, wenn Z für eine Abgangsgruppe steht, wobei dann ein weiteres Wasserstoffatom an das Kohlenstoffatom gebunden ist;
mit einer Verbindung der Formel III 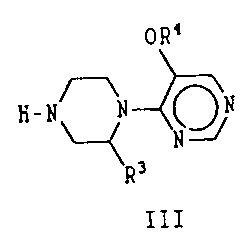 worin
R³ und R⁴ wie in Anspruch 1 definiert sind, umsetzt, unter der Voraussetzung, daß die Reaktion in Anwesenheit eines Reduktionsmittels erfolgt, wenn Z für Sauerstoff steht.

## Patentansprüche (Patentansprüche für folgende(n) Vertragsstaat(en) : ES)

1. Verfahren zur Herstellung einer Verbindung der folgenden Formel: 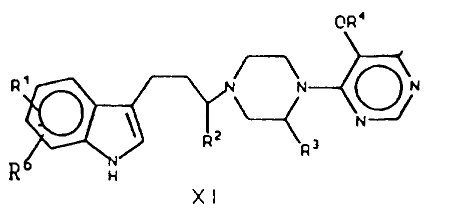 worin
R¹ ausgewählt ist unter Wasserstoff, Halogen, C₁-C₄-Alkoxy, Phenyl-C₁-C₄-alkoxy, Hydroxy und 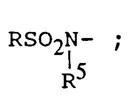
R², R³ und R⁵ unabhängig voneinander ausgewählt sind unter Wasserstoff und C₁-C₄-Alkyl;
_{R}⁴ für C1-C4-Alkyl steht;
R⁶ für Wasserstoff und Halogen steht,
oder eines pharmazeutisch akzeptablen Säureadditionssalzes und/oder Solvates und/oder Stereoisomers davon,
wobei man eine Verbindung der Formel XV 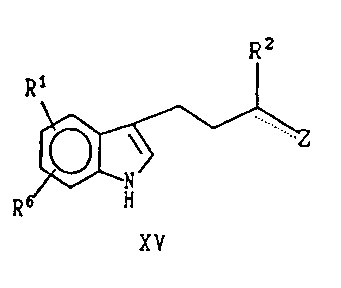 worin
R¹, R² und R⁶ wie oben definiert sind;
Z ausgewählt ist unter Sauerstoff oder einer Abgangsgruppe, die ausgewählt ist unter Tosyl, Mesyl, Halogenid, Sulfat und Phosphat; und
die gestrichelte Linie eine kovalente Bindung bedeutet,
wenn Z für Sauerstoff steht, oder keine Bindung bedeutet,
wenn Z für eine Abgangsgruppe steht, wobei dann ein weiteres Wasserstoffatom an das Kohlenstoffatom gebunden ist;
mit einer Verbindung der Formel III 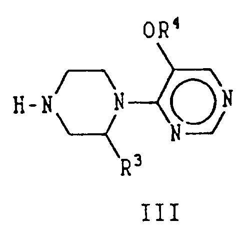 worin
R³ und R⁴ wie in Anspruch 1 definiert sind, umsetzt, unter der Voraussetzung, daß die Reaktion in Anwesenheit eines Reduktionsmittels erfolgt, wenn Z für Sauerstoff steht.

2. Verfahren nach Anspruch 1 zur Herstellung einer Verbindung der Formel 1 oder eines pharmaezeutisch akzeptablen Säureadditionssalzes davon 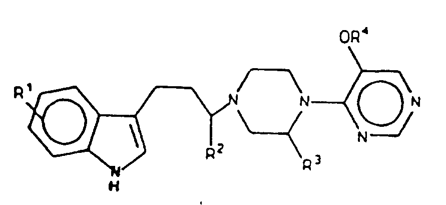 worin
R¹ ausgewählt ist unter Wasserstoff, Halogen oder 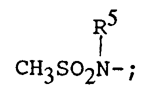
R², R³ und R⁵ unabhängig voneinander ausgewählt sind unter Wasserstoff und C1-C4-Alkyl; und
_{R}⁴ für C1-C4-Alkyl steht.

3. Verfahren nach Anspruch 1 zur Herstellung einer wie in Anspruch 1 oder 2 definierten Verbindung, wobei R¹ für Hydroxy steht.

4. Verfahren nach Anspruch 1 zur Herstellung einer wie in Anspruch 1 definierten Verbindung, die ausgewählt ist unter 4-(5-Ethoxy-4-pyrimidinyl)-1-[3-(1H-indol-3-yl)propyl]piperazin; 1-[3-(1H-Indol-3-yl)-propyl]-4-(5-methoxy-4-pyrimidinyl)piperazin; 1-[3-(5-Fluor-1H-indol-3-yl)propyl]-4-(5-methoxy-4-pyrimidinyl)piperazin; 1-[3-(5-Chlor-1H-indol-3-yl)propyl]-4-(5-methoxy-4-pyrimidinyl)-piperazin; 1-[3-(1H-IndoI-3-yl)propyl]-4-(5-methoxy-4-pyrimidinyl)-3-methylpiperazin; 1-[3-(1H-Indol-3-yl)butyl]-4-(5-methoxy-4-pyrimidinyl)piperazin; 1-[3-[5-(methylsulfonyl)-amino-1H-indol-3-yl]propyl]-4-(5-methoxy-4-pyrimidinyl)-piperazin; 1-[3-[5-(Methylsulfonyl)-amino-1H-indol-3-yl]propyl]-4-(5-methoxy-4-pyrimidinyl)-3-methylpiperazin; 1-[3-(5-Hydroxy-1H-indol-3-yl)propyl]-4-(5-m ethoxy-4-pyrimidinyl)-piperazin; 3-[3-[4-(5-Methoxy-4-pyrimidinyl)-1 -piperazinyl]pentyl]indol; 1-[3-[5-Methyl-sulfonyl)-methylamino-1H-indol-3-yl]propyl]-4-(5-methoxy-4-pyrimidinyl)piperazin; 1-[3-[5-Methylsulfonyl)-methylamino-1H-indol-3-yl]propyl]-4-(5-me- thoxy-4-pyrimidinyl)-3-methylpiperazin; 1-[3-[5-Ethylsulfonyl)-amino-1H-i ndol-3-yl]propyl]-4-(5-methoxy-4-pyrimidinyl)piperazin; 1-[3-[5-Ethylsulfonyl)-methylamino-1H-indol-3-yl]propyl]-4-(5-methoxy-4-pyrimidinyl)piperazin; 1,1-[3-[5-Ethylsulfonyl)-methylamino-1H-indol-3-yl]propyl]-4-(5-methoxy-4-pyrimidinyl)-3-methylpiperazin; 1-[3-[5-Ethylsulfonyl)-amino-1H-indol-3-yl]propyl]-4-(5-methoxy-4-pyrimidinyl)-3-methylpiperazin; 1-[3-(5-Ethoxy)-1H-indol-3-yl]propyl]-4-(5-methoxy-4-pyrimidinyl)piperazin; 1-[3-(5-Benzy- loxy-1H-indol-3-yl)propyl]-4-(5-methoxy-4-pyrimidinyl)piperazin; 1-[3-(5-Methoxy-1H-indol-3-yl)propyl]-4-(5-methoxy-4-pyrimidinyl)piperazin; 1-[3-(5,6-Difluor-1H-indol-3-yl)propyl]-4-(5-methoxy-4-pyrimidinyl)-piperazin; 1-[3-(4,7-Difluor-1H-indol-3-yl)propyl]-4-(5-methoxy-4-pyrimidinyl)piperazin; 1-[3-(5,7-Difluor-1H-indol-3-yl)propyl]-4-(5-methoxy-4-pyrimidinyl)piperazin, oder eines pharmazeutisch akzeptablen Säureadditionssalzes und/oder Solvates und/oder Stereoisomers davon.

5. Verfahren zur Herstellung einer pharmazeutischen Formulierung, wobei man eine wie in einem der Ansprüche 1 bis 4 definierte Verbindung mit einem oder mehreren pharmazeutisch akzeptablen Trägern, Exzipienten oder Verdünnungsmitteln vermischt.

6. Verfahren nach Anspruch 5, wobei die Formulierung in Dosiseinheitsform vorliegt.

7. Verfahren zur Herstellung einer pharmazeutischen Formulierung, wobei man 1-[3-(5-Fluor-1H-indol-3-yl)propyl]-4-(5-methoxy-4-pyrimidinyl)piperazin mit einem oder mehreren pharmazeutisch akzeptablen Trägern, Exzipienten oder Verdünnungsmitteln vermischt.

8. Verfahren nach Anspruch 7, wobei die Formulierung in Dosiseinheitsform vorliegt.

9. Verfahren nach einem der Ansprüche 5 bis 8, wobei die pharmazeutische Formulierung zur Verwendung als Antimigräneformulierung bestimmt ist.

## Patentansprüche (Patentansprüche für folgende(n) Vertragsstaat(en) : GR)

1. Verbindung der folgenden Formel: 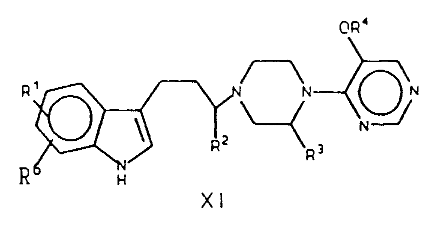 worin
R¹ ausgewählt ist unter Wasserstoff, Halogen, C₁-C₄-Alkoxy, Phenyl-C₁-C₄-alkoxy, Hydroxy und 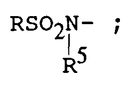
R², R³ und R⁵ unabhängig voneinander ausgewählt sind unter Wasserstoff und C₁-C₄-Alkyl;
R⁴ für C1-C4-Alkyl steht;
R⁶ für Wasserstoff und Halogen steht,
oder ein pharmazeutisch akzeptables Säureadditionssalz und/oder Solvat und/oder Stereoisomer davon.

2. Verbindung der Formel 1 oder ein pharmazeutisch akzeptables Säureadditionssalz davon 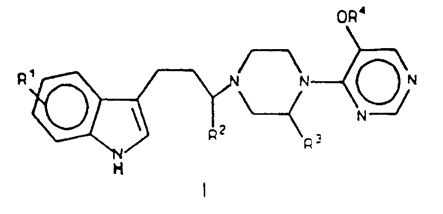 worin
R¹ ausgewählt ist unter Wasserstoff, Halogen oder 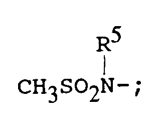
R², R³ und R⁵ unabhängig voneinander ausgewählt sind unter Wasserstoff und C₁-C₄-Alkyl; und
R⁴ für C1-C4-Alkyl steht.

3. Verbindung nach Anspruch 1 oder 2, wobei R¹ für Hydroxy steht.

4. Verbindung nach Anspruch 1, ausgewählt unter 4-(5-Ethoxy-4-pyrimidinyl)-1-[3-(1H-indol-3-yl)propyl]-piperazin; 1-[3-(1H-Indol-3-yl)propyl]-4-(5-methoxy-4-pyrimidinyl)-piperazin; 1-[3-(5-Fluor-1H-indol-3-yl)-propyl]-4-(5-methoxy-4-pyrimidinyl)piperazin; 1-[3-(5-Chlor-1H-indol-3-yl)propyl]-4-(5-methoxy-4-pyrimidinyl)piperazin; 1-[3-(lH-Indol-3-yl)propyl]-4-(5-methoxy-4-pyrimidinyl)-3-methylpiperazin; 1-[3-(lH-Indol-3-yl)butyl]-4-(5-methoxy-4-pyrimidinyl)piperazin; 1-[3-[5-(methylsulfonyl)-amino-1H-indol-3-yl]-propyl]-4-(5-methoxy-4-pyrimidinyl)piperazin; 1-[3-[5-(Methylsulfonyl)-amino-1H-indol-3-yl]propyl]-4-(5-methoxy-4-pyrimidinyl)-3-methylpiperazin; 1-[3-(5-Hydroxy-1H-indol-3-yl)propyl]-4-(5-methoxy-4-pyrimidinyl)-piperazin; 3-[3-[4-(5-Methoxy-4-pyrimidinyl)-1-piperazinyl]pentyl]indol; 1-[3-[5-Methylsulfonyl)-me- thylamino-1H-indol-3-yl]propyl]-4-(5-methoxy-4-pyrimidinyl)piperazin; 1-[3-[5-Methylsulfonyl)-methylami- no-1H-indol-3-yl]propyl]-4-(5-methoxy-4-pyrimidinyl)-3-methylpiperazin; 1-[3-[5-Ethylsulfonyl)-amino-1Hin dol-3-yl]propyl]-4-(5-methoxy-4-pyrimidinyl)piperazin; 1-[3-[5-Ethylsulfonyl)-methylamino-1H-indol-3-yl]propyl]-4-(5-methoxy-4-pyrimidinyl)piperazin; 1,1-[3-[5-Ethylsulfonyl)-methylamino-1H-indol-3-yl]-propyl]-4-(5-methoxy-4-pyrimidinyl)-3-met hylpiperazin; 1-[3-[5-Ethylsulfonyl)-amino-1H-indol-3-yl]-propyl]-4-(5-methoxy-4-pyrimidinyl)-3-methylpiperazin; 1-[3-(5-Ethoxy)-1H-indol-3-yl]propyl]-4-(5-me- thoxy-4-pyrimidinyl)piperazin; 1-[3-(5-Benzyloxy-1H-indol-3-yl)propyl]-4-(5-methoxy-4-pyrimidinyl)-piperazin; 1-[3-(5-Methoxy-1H-indol-3-yl)propyl]-4-(5-methoxy-4-pyrimidinyl)piperazin; 1-[3-(5,6-Difluor-1H-indol-3-yl)propyl]-4-(5-methoxy-4-pyrimidinyl)piperazin; 1-[3-(4,7-Difluor-1H-indol-3-yl)propyl]-4-(5-methoxy-4-pyrimidinyl)piperazin; 1-[3-(5,7-Difluor-1H-indol-3-yl)propyl]-4-(5-methoxy-4-pyrimidinyl)-piperazin, oder ein pharmazeutisch akzeptables Säureadditionssalz und/oder Solvat und/oder Stereoisomer davon.

5. 1-[3-(5-Fluor-1H-indol-3-yl)propyl]-4-(5-methoxy-4-pyrimidinyl)piperazin oder ein pharmazeutisch akzeptables Säureadditionssalz und/oder Solvat davon.

6. Verfahren zur Herstellung einer pharmazeutischen Formulierung, wobei man eine wie in einem der Ansprüche 1 bis 5 definierte Verbindung mit einem oder mehreren pharmazeutisch akzeptablen Trägern, Exzipienten oder Verdünnungsmitteln vermischt.

7. Verfahren nach Anspruch 6, wobei die Formulierung in Dosiseinheitsform vorliegt.

8. Verfahren zur Herstellung einer pharmazeutischen Formulierung, wobei man 1-[3-(5-Fluor-1H-indol-3-yl)propyl]-4-(5-methoxy-4-pyrimidinyl)piperazin mit einem oder mehreren pharmazeutisch akzeptablen Trägern, Exzipienten oder Verdünnungsmitteln vermischt.

9. Verfahren nach Anspruch 8, wobei die Formulierung in Dosiseinheitsform vorliegt.

10. Verbindung nach einem der Ansprüche 1 bis 5 zur Verwendung als Antimigränemittel.

11. 1-[3-(5-Fluor-1H-indol-3-yl)propyl]-4-(5-methoxy-4-pyrimidinyl)piperazin zur Verwendung als Antimigränemittel.

12. Verfahren nach einem der Ansprüche 6 bis 9, wobei die pharmazeutische Formulierung zur Verwendung als Antimigräneformulierung bestimmt ist.

13. Verfahren zur Herstellung einer Verbindung nach einem der Ansprüche 1 bis 5, wobei man eine Verbindung der Formel XV 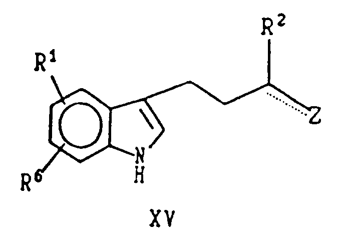 worin
R¹, R² und R⁶ wie in Anspruch 1 definiert sind;
Z ausgewählt ist unter Sauerstoff oder einer Abgangsgruppe, die ausgewählt ist unter Tosyl, Mesyl, Halogenid, Sulfat und Phosphat; und
die gestrichelte Linie eine kovalente Bindung bedeutet, wenn Z für Sauerstoff steht, oder keine Bindung bedeutet, wenn Z für eine Abgangsgruppe steht, wobei dann ein weiteres Wasserstoffatom an das Kohlenstoffatom gebunden ist;
mit einer Verbindung der Formel 111 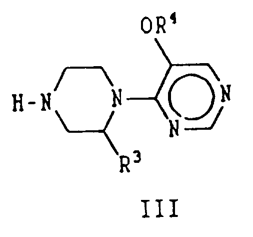 worin
R³ und R⁴ wie in Anspruch 1 definiert sind, umsetzt, unter der Voraussetzung, daß die Reaktion in Anwesenheit eines Reduktionsmittels erfolgt, wenn Z für Sauerstoff steht.

## Revendications (Revendications pour l'(les) Etat(s) contractant(s) suivant(s) : suivants : AT, BE, CH, DE, DK, FR, GB, IT, LI, LU, NL, SE)

1. Composé de la formule suivante : 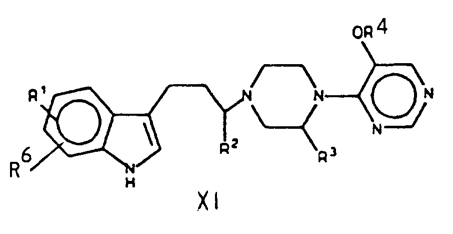 où
R¹ est choisi parmi l'hydrogène, un halogène, un alcoxy en C₁-C₄, un phényl -alcoxy en C₁-C₄, un hydroxy, et 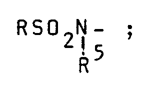
R², R³ et R⁵ sont indépendamment choisis parmi un hydrogène et un alkyle en C₁-C₄ ;
R⁴ est un alkyle en C₁ -C₄ ;
R⁶ est un hydrogène et un halogène
ou un stéréoisomère et/ou un solvate et/ou un sel d'addition d'acide pharmaceutiquement acceptable de celui-ci.

2. Composé de Formule 1 ou sel d'addition d'un acide pharmaceutiquement acceptable de celui-ci 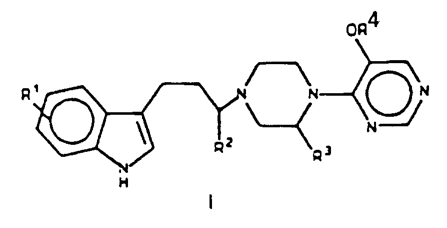 où
R¹ est choisi parmi un hydrogène, un halogène ; ou 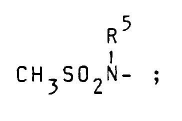
R², R³ et R⁵ sont indépendamment choisis parmi un hydrogène et un alkyle en C₁-C₄ ; et
R⁴ est un alkyle en C₁ -C₄.

3. Composé selon la revendication 1 ou 2, où R¹ est un hydroxyle.

4. Composé selon la revendication 1 choisi parmi la 4-(5-éthoxy-4-pyrimidinyl)-1-[3-(1H-indol-3-yl)propyl]-pipérazine ; 1-[3-(1H-indol-3-yl)propyl]-4-(5-méthoxy-4-pyrimidinyl)-pipérazine ; 1-[3-(5-fluoro-1H-indol-3-yl)propyl]-4-(5-méthoxy-4-pyrimidinyl)-pipérazine ; 1-[3-(5-chloro-1H-indol-3-yl)propyl]-4-(5-méthoxy-4-pyrimidinyl)-pipérazine ; 1-[3-(1H-indol-3-yl)propyl]-4-(5-méthoxy-4-pyrimidinyl)-3-méthyl-pipérazine ; 1-[3-(1H-indol-3-yl)butyl]-4-(5-méthoxy-4-pyrimidinyl)-pipérazine ; 1-[3-[5-(méthylsulfonyl)-amino-1H-in- dol-3-yl]propyl]-4-(5-méthoxy-4-pyrimidinyl)pipérazine, 1-[3-[5-(méthylsulfonyl)-amino-1H-indol-3-yl]-propyl]-4-(5-méthoxy-4-pyrimidinyl)-3-méthylpipérazine ; 1-[3-(5-hydroxy-1H-indol-3-yl)propyl]-4-(5-mé- thoxy-4-pyrimidinyl)-pipérazine ; 3-[3-[4-(5-méthoxy-4-pyrimidinyl)-1-pipérazinyl]pentyl]indole ; 1-[3-[5-méthylsulfonyl)-méthylamino-lH-indol-3-yl]propyl]-4-(5-méthoxy-4-pyrimidinyl)pipérazine ; 1-[3-[5-mé- thylsulfonyl)-méthylamino-1H-indol-3-yl]propyl]-4-(5-méthoxy-4-pyrimidinyl)-3-méthylpipérazine , 1-[3-[5-éthylsulfonyl)amino-1H-indol-3-yl]-propyl]-4-(5-méthoxy-4pyrimidinyl)pipérazine ; 1-[3-[5-éthyl-sulfo- nyl)-méthylamino-lH-indol-3-yl]propyl]-4-(5-méthoxy-4-pyrimidinyl)pipérazine ; 1,1-[3-[5-éthylsulfonyl)-méthylamino-lH-indol-3-yl]propyl]-4-(5-méthoxy-4-pyrimidinyl)-3-méthylpipérazine ; 1-[3-[5-éthylsulfo- nyl)amino-lH-indol-3-yl]propyl]-4-(5-méthoxy-4-pyrimidinyl)-3-méthylpipérazine ; 1-[3-(5-éthoxy)-1H-in- dol-3-yl]-4-(5-méthoxy-4-pyrimidinyl)pipérazine; 1-[3-(5-benzyloxy-1H-indol-3-yl)propyl]-4-(5-méthoxy-4-pyrimidinyl)pipérazine ; 1-[3-(5-méthoxy-1H-indol-3-yl)propyl]-4-(5-méthoxy-4-pyrimidinyl)pipérazine ; 1-[3-(5,6-difluoro-lH-indol-3-yl)propyl]-4-(5-méthoxy-4-pyrimidinyl)pipérazine ; 1-[3-(4,7-difluoro-lH-indol-3-yl)propyl]-4-(5-méthoxy-4-pyrimidinyl)pipérazine _{;} 1-[3-(5,7-difluoro-1H-indol-3-yl)-propyl]-4-(5-mé- thoxy-4-pyrimidinyl)pipérazine, ou un stéréoisomère et/ou solvate et/ou un sel d'addition d'un acide pharmaceutiquement acceptable de ceux-ci.

5. 1-[3-(5-fluoro-lH-indol-3-yl)propyl]-4-(5-méthoxy-4-pyrimidinyl)pipérazine ou sel d'addition d'un acide et/ou solvate pharmaceutiquement acceptable de celui-ci.

6. Formulation pharmaceutique qui comprend, en tant qu'ingrédient actif un composé, tel que revendiqué dans l'une quelconque des revendications 1-5, associé avec un ou plusieurs porteurs, excipients ou diluants pharmaceutiquement acceptables pour celui-ci.

7. Formulation pharmaceutique selon la revendication 6, où la formulation est sous forme de dosage unitaire.

8. Formulation pharmaceutique qui comprend,en tant qu'ingrédient actif de la 1-[3-(5-fluoro-1H-indol-3-yl)-propyl]-4-(5-méthoxy-4-pyrimidinyl)pipérazine associée avec un ou plusieurs porteurs, excipients ou diluants pharmaceutiquement acceptables de celle-ci.

9. Formulation pharmaceutique selon la revendication 8 où la formulation est sous forme de dosage unitaire.

10. Composé tel que revendiqué dans l'une quelconque des revendications 1-5 pour utilisation en tant qu'agent antimigraine.

11. 1-[3-(5-fluoro-1H-indol-3-yl)propyl]-4-(5-méthoxy-4-pyrimidinyl)pipérazine pour utilisation en tant qu'agent antimigraine.

12. Formulation pharmaceutique telle que revendiquée dans l'une quelconque des revendications 6 à 9 pour utilisation en tant que formulation antimigraine.

13. Procédé pour préparer un composé tel que revendiqué dans l'une quelconque des revendications 1-5 qui comprend la réaction d'un composé de Formule XV 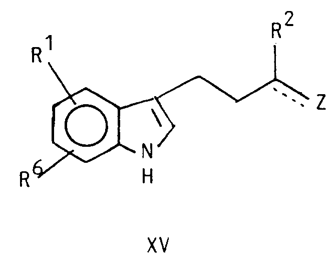 où R¹, R² et R⁶ sont tels que définis en revendication 1 ;
Z est choisi parmi un oxygène ou un fragment de groupe partant choisi parmi un tosyle, un méthyle, un halogénure, un sulfate et un phosphate ; et
la ligne en pointillé est soit une liaison covalente comme lorsque Z est un oxygène ou aucune liaison comme lorsque Z est un fragment de groupe partant et l'atome de carbone est ensuite lié à l'autre hydrogène ;
avec un composé de formule III 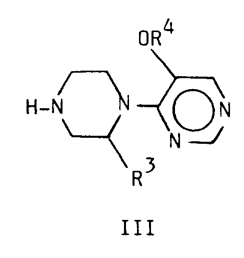
où R³ et R⁴ sont tels que définis en revendication 1, à la condition que lorsque Z est un oxygène, la réaction est menée en présence d'un agent réducteur.

## Revendications (Revendications pour l'(les) Etat(s) contractant(s) suivant(s) : suivant : ES)

1. Procédé pour préparer un composé de la formule suivante : 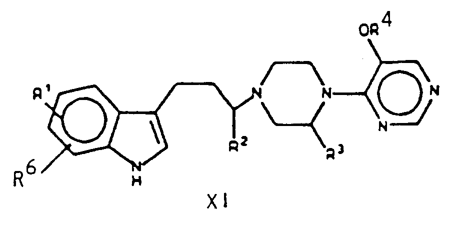 où
R¹ est choisi parmi un hydrogène, un halogène, un alcoxy en C₁-C₄, un phényl -alcoxy en C₁-C₄, un hydroxy, et 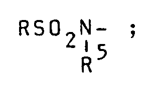
R², R³ et R⁵ sont indépendamment choisis parmi un hydrogène et un alkyle en C₁-C₄ ;
R⁴ est alkyle en Ci -C₄ ;
R⁶ est un hydrogène et un halogène
ou un stéréoisomère et/ou un solvate et/ou un sel d'addition d'un acide pharmaceutiquement acceptable de celui-ci, qui comprend la réaction d'un composé de Formule XV 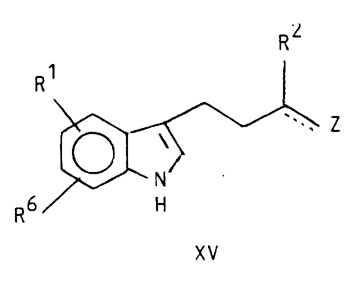 où R¹, R² et R⁶ sont tels que définis ci-dessus ;
Z est choisi parmi un oxygène au un fragment de groupe partant choisi parmi un tosyle, un mésyle, un halogénure, un sulfate et un phosphate ; et
la ligne en pointille est soit une liaison covalente comme lorsque Z est un oxygène ou pas de liaison lorsque Z est un fragment de groupe partant et l' atome de carbone est ensuite lié à l'autre hydrogène
avec un composé de formule III 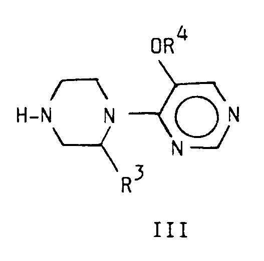
où R³ et R⁴ sont tels que définis ci-dessus,
à la condition que lorsque Z est un oxygène, la réaction est menée en présence d'un agent réducteur.

Procédé selon la revendication 1, pour préparer un composé de Formule 1 ou un sel d'addition d'un acide pharmaceutiquement acceptable de celui-ci 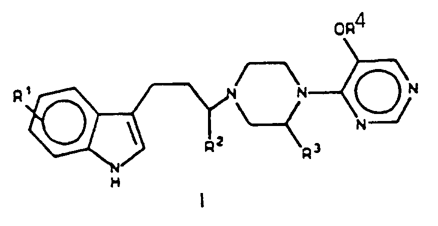 où
R¹ est choisi parmi un hydrogène, un halogène ; ou 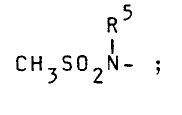
R², R³ et R⁵ sont indépendamment choisis parmi un hydrogène et un alkyle en C₁-C₄ ; et
R⁴ est un alkyle en C₁-C₄.

3. Procédé selon la revendication 1 pour préparer un composé tel que défini en revendication 1 ou 2, où R¹ est un hydroxyle.

4. Procédé selon la revendication 1 pour préparer un composé tel que défini en revendication 1, où ledit composé est choisi parmi la :
4-(5-éthoxy-4-pyrimidinyl)-1-[3-(lH-indol-3-yl)propyl]-pipérazine _{;} 1-[3-(lH-indol-3-yl)propyl]-4-(5-mé- thoxy-4-pyrimidinyl)-pipérazine ; ;1-[3-(5-fluoro-1H-indol-3-yl)propyl]-4-(5-méthoxy-4-pyrimidinyl)-pipérazi- ne ; 1-[3-(5-chloro-1H-indol-3-yl)propyl]-4-(5-méthoxy-4-pyrimidinyl)-pipérazine ; 1-[3-(1H-indol-3-yl)-propyl]-4-(5-méthoxy-4-pyrimidinyl)-3-méthyl-pipérazine ; 1-[3-(1H-indol-3-yl)butyl]-4-(5-méthoxy-4-pyrimidinyl)-pipérazine _{;} 1-[3-[5-(méthylsulfonyl)-amino-1H-indol-3-yl]propyl]-4-(5-méthoxy-4-pyrimidinyl)-pipérazine ; 1-[3-[5-(méthylsulfonyl)-amino-1H-indol-3-yl]propyl]-4-(5-méthoxy-4-pyrimidinyl)-3-méthylpi- pérazine ; 1-[3-(5-hydroxy-1H-indol-3-yl]propyl]-4-(5-méthoxy-4-pyrimidinyl)-pipérazine ; 3-[3-[4-(5-mé- thoxy-4-pyrimidinyl)-1-pipérazinyl]pentyl]indole _{;} 1-[3-[5-méthylsulfonyl)-méthylamino-lH-indol-3-yl]-propyl]-4-(5-méthoxy-4-pyrimidinyl)pipérazine _{;} 1-[3-[5-méthylsulfonyl)-méthylamino-1H-indol-3-yl]-propyl]-4-(5-méthoxy-4-pyrimidinyl)-3-méthylpipérazine ; 1-[3-[5-éthylsulfonyl)amino-1H-indol-3-yl]-pro- pyll-4-(5-méthoxy-4pyrimidinyl)pipérazine ; 1-[3-[5-éthylsulfonyl)-méthylamino-1H-indol-3-yl]propyl]-4-(5-méthoxy-4-pyrimidinyl)pipérazine _{;} 1,1-[3-[5-éthylsulfonyl)méthylamino-lH-indol-3-yl]propyl]-4-(5-mé- thoxy-4-pyrimidinyl)-3-méthyl-pipérazine , 1-[3-[5-éthylsulfonyl)amino-1H-indol-3-yl]propyl]-4-(5-mé- thoxy-4-pyrimidinyl)-3-méthylpipérazine _{;} 1 -[3-(5-éthoxy)-1 H-indol-3-yl]-4-(5-méthoxy-4-pyrimidinyl)-pipérazine; 1-[3-(5-benzyloxy-1H-indol-3-yl)propyl]-4-(5-méthoxy-4-pyrimidinyl)pipérazine ; 1-[3-(5-mé- thoxy-1H-indol-3-yl)propyl]-4-(5-méthoxy-4-pyrimidinyl)pipérazine _{;} 1-[3-(5,6-difluoro-1H-indol-3-yl)-propyl]-4-(5-méthoxy-4-pyrimidinyl)pipérazine ; 1-[3-(4,7-difluoro-1H-indol-3-yl)propyl]-4-(5-méthoxy-4-pyrimidinyl)pipérazine ; 1-[3-(5,7-difluoro-1H-indol-3-yl)-propyl]-4-(5-méthoxy-4-pyrimidinyl)pipérazine, ou un stéréoisomère et/ou un solvate et/ou un sel d'addition d'un acide pharmaceutiquement acceptable de ceux-ci.

5. Procédé pour préparer une formulation pharmaceutique qui comprend le mélange d'un composé tel que défini dans l'une quelconque des revendications 1 à 5 avec un ou plusieurs diluants, excipients ou porteurs pharmaceutiquement acceptables pour celle-ci.

6. Procédé selon la revendication 5, où la formulation est sous forme de dosage unitaire.

7. Procédé pour préparer une formulation pharmaceutique qui comprend le mélange de 1-[3-(5-fluoro-1H-indol-3-yl)-propyl]-4-(5-méthoxy-4-pyrimidinyl)pipérazine avec un ou plusieurs diluants, excipients ou porteurs pharmaceutiquement acceptables pour celle-ci

8. Procédé selon la revendication 7, où la formulation est sous forme de dosage unitaire.

9. Procédé selon l'une quelconque des revendications 6-9, où ladite formulation pharmaceutique est pour une utilisation en tant que formulation antimigraine.

## Revendications (Revendications pour l'(les) Etat(s) contractant(s) suivant(s) : suivant : GR)

1. Composé de la formule suivante : 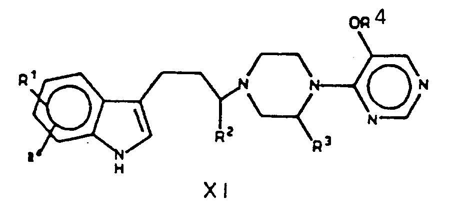 où
R¹ est choisi parmi l'hydrogène, un halogène, un alcoxy en C₁-C₄, un phényle-alcoxy en C₁-C₄, un hydroxy, et 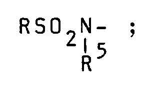
R², R³ et R⁵ sont indépendamment choisis parmi un hydrogène et un alkyle en C₁-C₄ ;
R⁴ est un alkyle en C₁ -C₄ ;
R⁶ est un hydrogène et un halogène
ou un stéréoisomère et/ou un solvate et/ou un sel d'addition d'acide pharmaceutiquement acceptable de celui-ci.

2. Composé de Formule 1 ou sel d'addition d'acide pharmaceutiquement acceptable de celui-ci 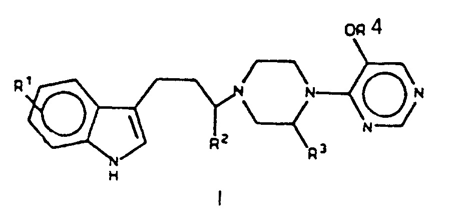 où
R¹ est choisi parmi un hydrogène, un halogène ; ou 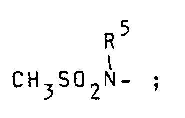
R², R³ et R⁵ sont indépendamment choisis parmi un hydrogène et un alkyle en C₁-C₄ ; et
R⁴ est un alkyle en C₁-C₄.

3. Composé selon la revendication 1 ou 2, où R¹ est un hydroxyle.

4. Composé selon la revendication 1 choisi parmi
4-(5-éthoxy-4-pyrimidinyl)-1-[3-(1H-indol-3-yl)propyl]-pipérazine _{;} 1-[3-(1H-indol-3-yl)propyl]-4-(5-mé- thoxy-4-pyrimidinyl)-pipérazine ; 1-[3-(5-fluoro-1 H-indol-3-yl)propyl]-4-(5-méthoxy-4-pyrimidinyl)-pipéra- zine ; 1-[3-(5-chloro-lH-indol-3-yl)propyl]-4-(5-méthoxy-4-pyrimidinyl)-pipérazine ; 1-[3-(lH-indol-3-yl)-propyl]-4-(5-méthoxy-4-pyrimidinyl)-3-méthyl-pipérazine ; 1-[3-(1H-indol-3-yl)butyl]-4-(5-méthoxy-4-pyrimidinyl)-pipérazine _{;} 1-[3-[5-(méthylsulfonyl)-amino-lH-indol-3-yl]propyl]-4-(5-méthoxy-4-pyrimidinyl)-pipérazine ; 1-[3-[5-(méthylsulfonyl)-amino-1H-indol-3-yl]propyl]-4-(5-méthoxy-4-pyrimidinyl)-3-méthylpi- pérazine ; 1-[3-(5-hydroxy-1H-indol-3-yl)propyl]-4-(5-méthoxy-4-pyrimidinyl)-pipérazine ; 3-[3-[4-(5-mé- thoxy-4-pyrimidinyl)-1-pipérazinyl]pentyl]indole _{;} 1-[3-[5-méthylsulfonyl)-méthylamino-1H-indol-3-yl]-propyl]-4-(5-méthoxy-4-pyrimidinyl)pipérazine _{;} 1-[3-[5-méthylsulfonyl)-méthylamino-1H-indol-3-yl]-propyl]-4-(5-méthoxy-4-pyrimidinyl)-3-méthylpipérazine ; 1-[3-[5-éthylsulfonyl)amino-1H-indol-3-yl]-propyl]-4-(5-méthoxy-4pyrimidinyl)pipérazine ; 1-[3-[5-éthylsulfonyl)-méthylamino-1H-indol-3-yl]propyl]-4-(5-méthoxy-4-pyrimidinyl)pipérazine ; 1,1-[3-[5-éthylsulfonyl)méthylamino-1H-indol-3-yl]propyl]-4-(5-mé- thoxy-4-pyrimidinyl)-3-méthylpipérazine : 1-[3-[5-éthylsulfonyl)amino-1-H-indol-3-yl]propyl]-4-(5-méthoxy-4-pyrimidinyl)-3-méthylpipérazine ; 1-[3-(5-éthoxy)-1H-indol-3-yl]-4-(5-méthoxy-4-pyrimidinyl)pipérazine; 1-[3-(5-benzyloxy-1-indol-3-yl)propyl]-4-(5-méthoxy-4-pyrimidinyl)pipérazine ; 1-[3-(5-méthoxy-1H-in- dol-3-yl)propyl]-4-(5-méthoxy-4-pyrimidinyl)pipérazine ; 1-[3-(5,6-difluoro-lH-indol-3-yl)propyl]-4-(5-mé- thoxy-4-pyrimidinyl)pipérazine _{;} 1-[3-(4,7-difluoro-1H-indol-3-yl)propyl]-4-(5-méthoxy-4-pyrimidinyl)-pipérazine ; 1-[3-(5,7-difluoro-lH-indol-3-yl)-propyl]-4-(5-méthoxy-4-pyrimidinyl)pipérazine, ou un stéréoisomère et/ou solvate et/ou un sel d'addition d'un acide pharmaceutiquement acceptable de ceux-ci.

5. 1-[3-(5-fluoro-lH-indol-3-yl)propyl]-4-(5-méthoxy-4-pyrimidinyl)pipérazine ou sel d'addition d'un acide et/ou solvate pharmaceutiquement acceptable de celui-ci.

6. Procédé pour préparer une formulation pharmaceutique qui comprend le mélange d'un composé tel que défini dans l'une quelconque des revendications 1 à 5 avec un ou plusieurs diluants, excipients ou porteurs pharmaceutiquement acceptables pour celle-ci.

7. Procédé selon la revendication 6, où la formulation est sous forme de dosage unitaire.

8. Procédé pour préparer une formulation pharmaceutique qui comprend le mélange de 1-[3-(5-fluoro-1H-indol-3-yl)-propyl]-4-(5-méthoxy-4-pyrimidinyl)pipérazine avec un ou plusieurs diluants, excipients ou porteurs pharmaceutiquement acceptables pour celle-ci.

9. Procédé selon la revendication 8, où la formulation est sous forme de dosage unitaire.

10. Composé tel que revendiqué dans l'une quelconque des revendications 1-5 pour utilisation en tant qu'agent antimigraine.

11. 1-[3-(5-fluoro-1H-indol-3-yl)propyl]-4-(5-méthoxy-4-pyrimidinyl)pipérazine pour utilisation en tant qu'agent antimigraine.

12. Procédé selon l'une quelconque des revendications 6-9, où ladite formulation pharmaceutique est pour utilisation en tant que formulation antimigraine.

13. Procédé pour préparer un composé tel que revendiqué dans l'une quelconque des revendications 1-5, qui comprend la réaction d'un composé de Formule XV 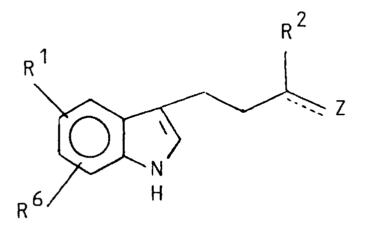
où R¹, R² et R⁶ sont tels que définis en revendication 1 ;
Z est choisi parmi un oxygène ou un fragment de groupe partant choisi parmi un tosyle, un mésyle, un halogénure, un sulfate et un phosphate ; et
la ligne en pointillé est soit une liaison covalente comme lorsque Z est un oxygène soit aucune liaison comme lorsque Z est un fragment de groupe partant et l'atome de carbone est ensuite lié à un autre hydrogène ;
avec un composé de Formule III 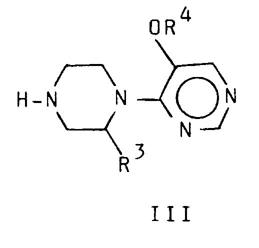
où R³ et R⁴ sont tels que définis en revendication 1,
avec la condition que si Z est un oxygène, la réaction est menée en présence d'un agent réducteur.
